# EUROPEAN PATENT SPECIFICATION

(11) **EP 2 558 584 B1**
(45) Date of publication and mention of the grant of the patent: **24.02.2016**
(21) Application number: 11768432.4
(22) Date of filing: 13.04.2011
(51) Int. Cl.: C12P 7/14, C12N 9/34, C12N 9/30

(54) **PROCESSES FOR PRODUCING FERMENTATION PRODUCTS**
VERFAHREN ZUR HERSTELLUNG VON GÄRUNGSPRODUKTEN
PROCÉDÉS POUR PRODUIRE DES PRODUITS DE FERMENTATION

(30) Priority: 14.04.2010 WO PCT/CN2010/071753
(43) Date of publication of application: 20.02.2013
(73) Proprietor: Novozymes A/S, 2880 Bagsvaerd (DK); Novozymes North America, Inc., Franklinton, North Carolina 27525 (US)
(72) Inventor: DEINHAMMER, Randall, North Carolina 27587 (US); COWARD-KELLY, Guillermo, Wake Forest North Carolina 27587 (US); LI, Ming, Beijing 100101 (CN); DUAN, Junxin, Beijing 100089 (CN); LIU, Zheng, Beijing 100017 (CN); FUKUYAMA, Shiro, Narashino-shi 275-0017 (JP); AYABE, Keiichi, Chiba-ken 263-0044 (JP)
(74) Representative: Shenker, Paul Dhan
(86) International application number: PCT/CN2011/072732
(87) International publication number: WO 2011/127820

(56) References cited:
- WO-A2-2010/008841
- WO-A2-2010/008841
- US-A1- 2003 027 290
- US-A1- 2006 270 007
- US-A1- 2009 203 101
- US-B2- 7 371 546
- YOSHIKI YAMASAKI ET AL: "Purification and Properties of Two Forms of Glucoamylase from Penicillium oxalicum", AGRICULTURAL & BIOLOGICAL CHEMISTRY,, vol. 41, no. 5, 1 May 1977 (1977-05-01), pages 755-762, XP001316482,
- YAMASAKI Y ET AL: "PROPERTIES OF 2 FORMS OF GLUCO AMYLASE FROM PENICILLIUM-OXALICUM", AGRICULTURAL AND BIOLOGICAL CHEMISTRY, JAPAN SOC. FOR BIOSCIENCE, BIOTECHNOLOGY AND AGROCHEM, TOKYO, JP, vol. 41, no. 8, 1 January 1977 (1977-01-01), pages 1443-1450, XP009173548, ISSN: 0002-1369
- SAHA B C ET AL: "MICROBIAL GLUCOAMYLASES: BIOCHEMICAL AND BIOTECHNOLOGICAL FEATURES", STARKE - STARCH, WILEY-VCH VERLAG, WEINHEIM, DE, vol. 41, no. 2, 1 February 1989 (1989-02-01), pages 57-64, XP000024425, ISSN: 0038-9056

## Description

### FIELD OF THE INVENTION

The present invention relates to processes for producing fermentation products from starch-containing material.

### BACKGROUND OF THE INVENTION

Production of fermentation products, such as ethanol, from starch-containing material is well-known in the art. Industrially mainly two different kinds of processes are used today. The most commonly used process, often referred to as the "conventional process" includes liquefying gelatinized starch at high temperature using a bacterial alpha-amylase followed by simultaneous saccharification and fermentation carried out in the presence of a glucoamylase and a fermenting organism. Another well-known process type often referred to as the "raw starch hydrolysis"-process ("RSH"-process) includes simultaneously saccharifying and fermenting granular starch below the initial gelatinization temperature typically in the presence of an acid fungal alpha-amylase and a glucoamylase.

WO 2010/008841 discloses processes for producing fermentation products from starch-containing material comprising simultaneously saccharifying and fermenting starch-containing material using a carbohydrate-source generating enzyme, in particular a glucoamylase, and a fermenting organism at a temperature below the initial gelatinization temperature of said starch-containing material in the presence of a metallo protease.

Despite significant improvement of fermentation product production processes over the past decade a significant amount of residual starch material is not converted into the desired fermentation product, such as ethanol. At least some of the unconverted residual starch materials, e.g., sugars and dextrins, are in the form of non-fermentable Maillard products.

Therefore, there is still a desire and need for providing processes for producing fermentation products, such as ethanol, from starch-containing material that can provide a higher fermentation product yield compared to a conventional process.

### SUMMARY OF THE INVENTION

The present invention relates to processes of producing fermentation products, such as ethanol from starch-containing material using a fermenting organism.

In the first aspect the invention relates to processes for producing fermentation products, such as ethanol, from starch-containing material comprising the steps of:
i) liquefying the starch-containing material using an alpha-amylase;
ii) saccharifying using a glucoamylase having at least 80% identity to the mature polypeptide shown in SEQ ID NO: 9;
iii) fermenting using a fermenting organism.

### DETAILED DESCRIPTION OF THE INVENTION

The present invention relates to processes of producing fermentation products, such as ethanol, from starch-containing material using a fermenting organism.

The inventors have found that a process of the invention has a number of advantages compared to a conventional process. As shown in, e.g., Example 5, a process where a glucoamylase, such as the *Penicillium oxalicum* glucoamylase, having a heat stability at 70°C, pH 5.3, of at least 70% relative activity (determined as described in Example 4) is added during pre-saccharification (pH 5.0 at 70°C) results in a higher glucose level compared to adding the same glucoamylase during liquefaction (pH 4.5 at 85°C). When the same glucoamylase is added during liquefaction it does not perform well in absence of additional glucoamylase added during fermentation, giving rise to a significantly slower fermentation rate and lower ethanol titer. However, when added during pre-saccharification the glucoamylase performs well in the absence of additional glucoamylase being added during fermentation with the potential to lower the enzyme protein need. Furthermore, due to the higher thermostability of the enzyme used the enzyme dose may be reduced. A process of the invention requires limited changes to existing process and process equipment and thus limited capital investment.

In the first aspect the invention relates to processes for producing fermentation products, such as ethanol, from starch-containing material comprising the steps of:
i) liquefying the starch-containing material using an alpha-amylase;
ii) saccharifying using a glucoamylase having at least 80% identity to the mature polypeptide shown in SEQ ID NO: 9;
iii) fermenting using a fermenting organism.

### Liquefaction Step i)

Liquefaction step i) may be carried out in the presence of an alpha-amylase at conditions well-known in the art.

In an embodiment, the process of the invention further comprises, prior to liquefaction step i), the steps of:
a) reducing the particle size of the starch-containing material;
b) forming a slurry comprising the starch-containing material and water.

The starch-containing starting material, such as whole grains, may be reduced in particle size, e.g., by milling, in order to open up the structure and allowing for further processing. Generally there are two types of milling processes: wet and dry milling. In dry milling whole kernels are milled and used. Wet milling gives a good separation of germ and meal (starch granules and protein) and is often applied at locations where the starch hydrolysate is used in production of, e.g., syrups. Both dry and wet milling is well-known in the art. According to the invention dry milling is preferred. In an embodiment the particle size is reduced to between 0.05 to 3.0 mm, preferably 0.1-0.5 mm, or so that at least 30%, preferably at least 50%, more preferably at least 70%, even more preferably at least 90% of the starch-containing material fit through a sieve with a 0.05 to 3.0 mm screen, preferably 0.1-0.5 mm screen. In another embodiment at least 50%, preferably at least 70%, more preferably at least 80%, especially at least 90% of the starch-containing material fit through a sieve with # 6-8 screen.

The aqueous slurry may contain from 10-55 w/w-% dry solids (DS), preferably 25-45 w/w-% dry solids (DS), more preferably 30-40 w/w-% dry solids (DS) of starch-containing material.

The slurry is heated to above the gelatinization temperature. In an embodiment the slurry is heated to above the gelatinization temperature, preferably at between 80-90°C, pH 4.5-4.8 for around 15-60 minutes.

In an embodiment the slurry is jet-cooked at a temperature between 95-140°C, preferably 105-125°C, for 1-15 minutes, preferably for 3-10 minute, especially around 5 minutes, before step i).

In an embodiment the pH during liquefaction is between about 4.0 and 7.0, such as between 4.5 and 6.0, such as around 5.8 or between 4.5-4.8.

In an embodiment the alpha-amylase is a bacterial alpha-amylase or a fungal alpha-amylase.

In a preferred embodiment the alpha-amylase is a *Bacillus* alpha-amylase. Suitable alpha-amylases can be found in the "Alpha-Amylase Present and/or Added During Liquefaction"-section below.

In a preferred embodiment the alpha-amylase is a thermostable alpha-amylase. In a specific embodiment the bacterial alpha-amylase is a *Bacillus* alpha-amylase, preferably derived from *Bacillus stearothermophilus* alpha-amylase (sometimes referred to as *Geobacillus stearothermophilus*) or a variant thereof comprising a double deletion such as: R179* and G180* or I181*+G182*, especially I181*+G182*+NI93F. The bacterial alpha-amylase is typically added in an amount between 0.0005-5 KNU per g DS, preferably between 0.001-1 KNU per g DS, such as around 0.06 KNU per g DS, or 0.0005-5 KNU(S) per g DS, preferably between 0.001-1 KNU(S) per g DS, such as around 0.060 KNU(S) per g DS if it is a *Bacillus stearothermophilus* alpha-amylase.

A protease may optionally be present during liquefaction step i), saccharification step ii) and/or fermentation step iii), such as SSF. In an embodiment the protease is a thermostable protease added during liquefaction step i). In an embodiment the protease, such as a thermostable protease, is added during saccharification step ii) and/or fermentation step iii), such as during SSF. Examples of proteases, including thermostable proteases, can be found in the "Protease Added During A Process Step Of The Invention"-section below.

A pullulanase (including amylopullulanases) may optionally be present during liquefaction step i), saccharification step ii) and/or fermentation step iii), such as during SSF. In an embodiment the pullulanse is a thermostable pullulanase added during liquefaction step i). Examples of pullulanases, including thermostable pullulanases, can be found in the "Pullulanase Present and/or Added During A Process Step Of The Invention"-section below.

### Saccharification Step ii) and/or Fermentation Step iii)

Saccharification step ii) and fermentation step iii) may be carried out separately (sequentially) or simultaneously.

In a preferred embodiment saccharification is carried out as a pre-saccharification followed by a separate fermentation step iii) or a step where saccharification is completed during fermentation (SSF), i.e., pre-saccharification is followed by simultaneous saccharification and fermentation (SSF). In one embodiment the pre-saccharification step is completed before fermentation. In an embodiment no additional carbohydrate-source generating enzyme, such as glucoamylase, is added during fermentation step iii), i.e., after saccharification or pre-saccharification.

Pre-saccharification is done at conditions that are more suitable for the enzyme(s) added (e.g., conditions suitable for the carbohydrate-source generating enzyme, such as glucoamylase), than at fermentation conditions (i.e., adapted to the fermenting organism) Fermentation is typically carried out at a significantly lower temperature, e.g., 25-40°C, such as around 32°C, while saccharification or pre-saccharification is typically carried out as indicated below.

When saccharification step ii) is carried out as a pre-saccharification step or separate saccharification step the pH may be in the range from 4-7, preferably from pH 4.5-5. In an embodiment saccharification step ii) is carried out at a temperature from 50-80°C, preferably from 60-75°C, such as around 70°C. In an embodiment saccharification step ii) is carried out for 10 minutes to 6 hours, preferably 30 minutes to 3 hours, such as 90 minutes to 150 minutes.

Saccharification step ii) and fermentation step iii) may in an embodiment be carried out simultaneously. This is widely used in industrial scale fermentation product production processes, such as ethanol production processes. When doing SSF the saccharification step ii) and the fermentation step iii) are carried out simultaneously. There is no holding stage for the saccharification, meaning that a fermenting organism, such as yeast, and enzyme(s), may be added together. SSF or a separate fermentation step iii) are according to the invention typically carried out at a temperature from 25°C to 40°C, such as from 28°C to 35°C, such as from 30°C to 34°C, preferably around about 32°C. In an embodiment fermentation step iii) or SSF is ongoing for 6 to 120 hours, in particular 24 to 96 hours. In an embodiment the pH is between 3.5 and 5, in particular between 3.8 and 4.3.

The glucoamylase used in saccharification step ii) is a glycoamylase preferably derived from *Penicillium oxalicum* having at least 80% identity to the mature polypeptide shown in SEQ ID NO: 9.

According to the invention the glucoamylase is added in an amount of 0.0001-20 AGU/g DS, preferably 0.001-10 AGU/g DS, especially between 0.01-5 AGU/g DS, such as 0.1-2 AGU/g DS.

### Fermentation Medium

"Fermentation media" or "fermentation medium" which refers to the environment in which fermentation is carried out and which includes the fermentation substrate, that is, the carbohydrate source that is metabolized by the fermenting organism. The fermentation medium may comprise nutrients and growth stimulator(s) for the fermenting organism(s). Nutrient and growth stimulators are widely used in the art of fermentation and include nitrogen sources, such as ammonia; urea, vitamins and minerals, or combinations thereof.

### Fermenting Organisms

The term "fermenting organism" may refer to any organism, including bacterial and fungal organisms, suitable for use in a fermentation process and capable of producing the desired fermentation product. Especially suitable fermenting organisms are able to ferment, i.e., convert, sugars, such as glucose or maltose, directly or indirectly into the desired fermentation product, such as ethanol. Examples of fermenting organisms include fungal organisms, such as yeast. Preferred yeast includes strains of *Saccharomyces* spp., in particular, *Saccharomyces cerevisiae*.

In one embodiment the fermenting organism is added to the fermentation medium so that the viable fermenting organism, such as yeast, count per mL of fermentation medium is in the range from 10⁵ to 10¹², preferably from 10⁷ to 10¹⁰, especially about 5x10⁷.

Commercially available yeast includes, e.g., RED STAR™ and ETHANOL RED™ yeast (available from Fermentis/Lesaffre, USA), FALI (available from Fleischmann's Yeast, USA), SUPERSTART and THERMOSACC™ fresh yeast (available from Ethanol Technology, WI, USA), BIOFERM AFT and XR (available from NABC - North American Bioproducts Corporation, GA, USA), GERT STRAND (available from Gert Strand AB, Sweden), and FERMIOL (available from DSM Specialties).

### Starch-Containing Materials

Any suitable starch-containing material may be used according to the present invention. The starting material is generally selected based on the desired fermentation product. Examples of starch-containing materials, suitable for use in a process of the invention, include whole grains, corn, wheat, barley, rye, milo, sago, cassava, tapioca, sorghum, rice, peas, beans, or sweet potatoes, or mixtures thereof or starches derived there from, or cereals. Contemplated are also waxy and non-waxy types of corn and barley.

### Fermentation Products

The term "fermentation product" means a product produced by a process including a fermentation step using a fermenting organism. Fermentation products contemplated according to the invention include alcohols (e.g., ethanol, methanol, butanol); organic acids (e.g., citric acid, acetic acid, itaconic acid, lactic acid, succinic acid, gluconic acid); ketones (e.g., acetone); amino acids (e.g., glutamic acid); gases (e.g., H₂ and CO₂); antibiotics (e.g., penicillin and tetracycline); enzymes; vitamins (e.g., riboflavin, B₁₂, beta-carotene); and hormones. In a preferred embodiment the fermentation product is ethanol, e.g., fuel ethanol; drinking ethanol, i.e., potable neutral spirits; or industrial ethanol or products used in the consumable alcohol industry (e.g., beer and wine), dairy industry (e.g., fermented dairy products), leather industry and tobacco industry. Preferred beer types comprise ales, stouts, porters, lagers, bitters, malt liquors, happoushu, high-alcohol beer, low-alcohol beer, low-calorie beer or light beer. Preferred fermentation processes used include alcohol fermentation processes. In an embodiment the fermentation product obtained according to the invention is ethanol. In an embodiment the fermentation product is fuel ethanol, which is typically blended with gasoline. In an embodiment the fermentation product is potable ethanol.

### Recovery

Subsequent to fermentation the fermentation product may be separated from the fermentation medium. The slurry may be distilled to extract the desired fermentation product. Alternatively the desired fermentation product may be extracted from the fermentation medium by micro or membrane filtration techniques. The fermentation product may also be recovered by stripping or other method well known in the art.

### Alpha-Amylase Present and/or Added During Liquefaction

According to the process of the invention any alpha-amylase may be used. In a preferred embodiment the alpha-amylase is of either bacterial or fungal origin. In a preferred embodiment the alpha-amylase is a *Bacillus* alpha-amylase.

In another embodiment the alpha-amylase is an acid alpha-amylase, such as especially an acid fungal alpha-amylase.

The term "acid alpha-amylase" means an alpha-amylase (E.C. 3.2.1.1) which added in an effective amount has an activity optimum at a pH in the range of 3 to 7, preferably from 3.5 to 6, or more preferably from 4-5.

### Bacterial Alpha-Amylases

According to the invention the bacterial alpha-amylase is preferably derived from the genus *Bacillus.*

In a preferred embodiment the *Bacillus* (or *Geobacillus*) alpha-amylase is derived from a strain of *Bacillus licheniformis, Bacillus amyloliquefaciens, Bacillus subtilis* or *Bacillus stearothermophilus (Geobacillus stearothermophilus),* but may also be derived from other *Bacillus* sp. Specific examples of contemplated alpha-amylases include the *Bacillus licheniformis* alpha-amylase shown in SEQ ID NO: 4 in WO 99/19467, the *Bacillus amyloliquefaciens* alpha-amylase shown in SEQ ID NO: 5 in WO 99/19467 and the *Bacillus stearothermophilus* alpha-amylase shown in SEQ ID NO: 3 in WO 99/19467. In an embodiment the alpha-amylase may be an enzyme having a degree of identity of at least 60%, preferably at least 70%, more preferred at least 80%, even more preferred at least 90%, such as at least 95%, at least 96%, at least 97%, at least 98% or at least 99% to any of the sequences shown in SEQ ID NOS: 1, 2 or 3, respectively, in WO 99/19467.

The *Bacillus* alpha-amylase may also be a variant and/or a hybrid, especially one described in any of WO 96/23873, WO 96/23874, WO 97/41213, WO 99/19467, WO 00/60059, and WO 02/10355. Specifically contemplated alpha-amylase variants are disclosed in US patent nos. 6,093,562, 6,297,038 or US patent no. 6,187,576 and include *Bacillus stearothermophilus* alpha-amylase (i.e., BSG alpha-amylase) variants having a deletion of one or two amino acid in positions R179 to G182, preferably a double deletion disclosed in WO 1996/023873 - see e.g., page 20, lines 1-10, preferably corresponding to delta(181-182) compared to the wild-type BSG alpha-amylase amino acid sequence set forth in SEQ ID NO: 3 disclosed in WO 99/19467 or deletion of amino acids R179 and G180 using SEQ ID NO: 3 in WO 99/19467 for numbering. Even more preferred are *Bacillus* alpha-amylases, especially *Bacillus stearothermophilus* alpha-amylase, which have a double deletion corresponding to delta(181-182) and further comprise a N193F substitution (also denoted I181* + G182* + N193F) compared to the wild-type BSG alpha-amylase amino acid sequence set forth in SEQ ID NO:3 disclosed in WO 99/19467.

In an embodiment the alpha-amylase is a *Bacillus stearothermophilus* alpha-amylase comprising a substitution in position S242, preferably S242A,E or Q, especially S242Q, or any of the substitutions claimed in US patent No. 7,713,723 (Novozymes), WO 2010/036515 (Danisco) or US patent No. 7,541,026 (Danisco).

### Bacterial Hybrid Alpha-Amylases

A hybrid alpha-amylase specifically contemplated comprises 445 C-terminal amino acid residues of the *Bacillus licheniformis* alpha-amylase (shown in SEQ ID NO: 4 of WO 99/19467) and the 37 N-terminal amino acid residues of the alpha-amylase derived from *Bacillus amyloliquefaciens* (shown in SEQ ID NO: 5 of WO 99/19467), with one or more, especially all, of the following substitution:
G48A+T49I+G107A+H156Y+A181T+N190F+I201F+A209V+Q264S (using the *Bacillus licheniformis* numbering in SEQ ID NO: 4 of WO 99/19467). Also preferred are variants having one or more of the following mutations (or corresponding mutations in other Bacillus alpha-amylase backbones): H154Y, A181T, N190F, A209V and Q264S and/or deletion of two residues between positions 176 and 179, preferably deletion of E178 and G179 (using the SEQ ID NO: 5 numbering of WO 99/19467).

In an embodiment the bacterial alpha-amylase is dosed in an amount of 0.0005-5 KNU per g DS, preferably 0.001-1 KNU per g DS, such as around 0.050 KNU per g DS, or 0.0005-5 KNU(S) per g DS, preferably between 0.001-1 KNU(S) per g DS, such as around 0.060 KNU(S) per g DS if it is a *Bacillus stearothermophilus* alpha-amylase.

### Commercial Alpha-Amylase Products

Preferred commercial compositions comprising alpha-amylase include MYCOLASE™ from DSM (Gist Brocades), BAN™, TERMAMYL™ SC, FUNGAMYL™, LIQUOZYME™ X, LIQUOZYME™ SC, LIQUOZYMET™ SCDS and SAN™ SUPER, SAN™ EXTRA L (Novozymes A/S) and CLARASE™ L-40,000, DEX-LOT™, SPEZYME® FRED-L, SPEZYME® HPA, SPEZYME® ALPHA, SPEZYME® XTRA, SPEZYME® AA, SPEZYME® DELTA AA , and GC358, SPEZYME RSL (Danisco); FUELZYME™-LF (Verenium Inc), and the acid fungal alpha-amylase sold under the trade name SP288 (available from Novozymes A/S, Denmark).

### Thermostable Alpha-Amylase at Low pH

According to the invention the alpha-amylase used in step i) may be a thermostable alpha-amylase at low pH (i.e., pH 4.5-5.0). According to the invention the alpha-amylase has high activity toward starch solubilisation in liquefaction at pH 4.5 to 5.0 and high thermostability at pH 4.5-5.0 and 80-90°C, preferably 4.5-4.8, 85°C.

More specifically the alpha-amylase used in liquefaction step i) may preferably have a T½ (min) at pH 4.5, 85°C, 0.12 mM CaCl₂ of at least 10.

In a preferred embodiment T½ (min) at pH 4.5, 85°C, 0.12 mM CaCl₂, is at least 15, such as at least 20, such as at least 25, such as at least 30, such as at least 40, such as at least 50, such as at least 60, such as between 10-70, such as between 15-70, such as between 20-70, such as between 25-70, such as between 30-70, such as between 40-70, such as between 50-70, such as between 60-70.

In an embodiment of the invention the alpha-amylase is an bacterial alpha-amylase, preferably derived from the genus *Bacillus,* especially a strain of *Bacillus stearothermophilus,* in particular the *Bacillus stearothermophilus* as disclosed in WO 99/019467 as SEQ ID NO: 3 (SEQ ID NO: 1 herein) with the double deletion I181 + G182 and substitution N193F, further comprising the following mutations:
- V59A+Q89R+G112D+E129V+K177L+R179E+K220P+N224L+Q254S;
- V59A+Q89R+E129V+K177L+R179E+H208Y+K220P+N224L+Q254S;
- V59A+Q89R+E129V+K177L+R179E+K220P+N224L+Q254S+D269E+D281N;
- V59A+Q89R+E129V+K177L+R179E+K220P+N224L+Q254S+I270L;
- V59A+Q89R+E129V+K177L+R179E+K220P+N224L+Q254S+H274K;
- V59A+Q89R+E129V+K177L+R179E+K220P+N224L+Q254S+Y276F;
- V59A+E129V+R157Y+K177L+R179E+K220P+N224L+S242Q+Q254S;
- V59A+E129V+K177L+R179E+H208Y+K220P+N224L+S242Q+Q254S;
- 59A+E129V+K177L+R179E+K220P+N224L+S242Q+Q254S;
- V59A+E129V+K177L+R179E+K220P+N224L+S242Q+Q254S+H274K;
- V59A+E129V+K177L+R179E+K220P+N224L+S242Q+Q254S+Y276F;
- V59A+E129V+K177L+R179E+K220P+N224L+S242Q+Q254S+D281N;
- V59A+E129V+K177L+R179E+K220P+N224L+S242Q+Q254S+M284T;
- V59A+E129V+K177L+R179E+K220P+N224L+S242Q+Q254S+G416V;
- V59A+E129V+K177L+R179E+K220P+N224L+Q254S;
- V59A+E129V+K177L+R179E+K220P+N224L+Q254S+M284T;
- A91L+M961+E129V+K177L+R179E+K220P+N224L+S242Q+Q254S;
- E129V+K177L+R179E;
- E129V+K177L+R179E+K220P+N224L+S242Q+Q254S;
- E129V+K177L+R179E+K220P+N224L+S242Q+Q254S+Y276F+L427M;
- E129V+K177L+R179E+K220P+N224L+S242Q+Q254S+M284T;
- E129V+K177L+R179E+K220P+N224L+S242Q+Q254S+N376*+I377*;
- E129V+K177L+R179E+K220P+N224L+Q254S;
- E129V+K177L+R179E+K220P+N224L+Q254S+M284T;
- E129V+K177L+R179E+S242Q;
- E129V+K177L+R179V+K220P+N224L+S242Q+Q254S;
- K220P+N224L+S242Q+Q254S; or
- M284V.

### Proteases Added During A Process Step Of The Invention

According to the invention a protease may optionally be present and/or added during a process step of the invention. Due to the relative high temperature generally used during liquefaction it is preferred to add a thermostable protease during liquefaction step i) together with an alpha-amylase as described above. In other embodiment a protease is added during saccharification step ii) and/or fermentation step iii), such as SSF.

According to the present invention the starch-containing material may be treated with a protease, such as a thermostable protease, of any origin. Suitable proteases may be of fungal, bacterial, including filamentous fungi and yeast, and plant origin.

According to the invention a peptidase and other protein degrading enzymes are encompassed by the term "protease". In an embodiment the protease is an endo-protease and/or an exo-protease.

In an embodiment the protease, such as thermostable protease, is an acidic protease, i.e., a protease characterized by the ability to hydrolyze proteins under acidic conditions below pH 7, e.g., at a pH between 2-7. In an embodiment the acidic protease has an optimum pH in the range from 2.5 and 3.5 (determined on high nitrogen casein substrate at 0.7% w/v at 37°C) and a temperature optimum between 5 to 50°C at an enzyme concentration of 10 mg/mL at 30°C for one hour in 0.1 M pipera-zine/acetate/glycine buffer).

In another embodiment the protease, or thermostable protease, is an alkaline protease, i.e., a protease characterized by the ability to hydrolyze proteins under alkaline conditions above pH 7, e.g., at a pH between 7-11. In an embodiment the alkaline protease is derived from a strain of *Bacillus,* preferably *Bacillus licheniformis.* In an embodiment the alkaline protease has an optimum temperature in the range from 7 and 11 and a temperature optimum around 70°C determined at pH 9.

In another embodiment the protease, or thermostable protease, is a neutral protease, i.e., a protease characterized by the ability to hydrolyze proteins under conditions between pH 5 and 8. In an embodiment the alkaline protease is derived from a strain of *Bacillus,* preferably *Bacillus amyloliquefaciens.* In an embodiment the alkaline protease has an optimum pH in the range between 7 and 11 (determined at 25°C, 10 minutes reaction time with an enzyme concentration of 0.01-0.2 AU/L) and a temperature optimum between 50°C and 70°C (determined at pH 8.5, 10 minutes reaction time and 0.03-0.3 AU/L enzyme concentration.

Suitable plant proteases may be derived from barley.

Suitable bacterial proteases include *Bacillus* proteases derived from *Bacillus amyloli-quefaciens* and *Bacillus licheniformis.* Suitable filamentous bacterial proteases may be derived from a strain of *Nocardiopsis,* preferably *Nocardiopsis prasina* NRRL 18262 protease (or *Nocardiopsis* sp. 10R) and *Nocardiopsis dassonavilla* NRRL 18133 *(Nocardiopsis dassonavilla* M58-1) both described in WO 1988/003947 (Novozymes).

Suitable acid fungal proteases include fungal proteases derived from *Aspergillus, Mucor, Rhizomucor, Rhizopus, Candida, Coriolus, Endothia, Enthomophtra, Irpex, Penicillium, Sclerotium, Thermoaccus,* and *Torulopsis.* Especially contemplated are proteases derived from *Aspergillus niger* (see, e.g., Koaze et al., (1964), Agr. Biol. Chem. Japan, 28, 216), *Aspergillus saitoi* (see, e.g., Yoshida, (1954) J. Agr. Chem. Soc. Japan, 28, 66), Aspergillus awamori (Hayashida et al., (1977) Agric. Biol. Chem., 42(5), 927-933, *Aspergillus aculeatus* (WO 95/02044), or *Aspergillus oryzae;* proteases from *Mucor pusillus* or *Mucor miehei* disclosed in US patent no. 4,357,357 and US patent no. 3,988,207; and *Rhizomucor mehei* or *Rhizomucor pusillus* disclosed in, e.g., WO 94/24880.

Aspartic acid proteases are described in, for example, Hand-book of Proteolytic En-zymes, Edited by A.J. Barrett, N.D. Rawlings and J.F. Woessner, Aca-demic Press, San Diego, 1998, Chapter 270). Suitable examples of aspartic acid protease include, e.g., those disclosed in R.M. Berka et al. Gene, 96, 313 (1990)); (R.M. Berka et al. Gene, 125, 195-198 (1993)); and Gomi et al. Biosci. Biotech. Biochem. 57, 1095-1100 (1993).

Commercially available products include ALCALASE®, ESPERASE™, NEUTRASE®, RENILASE®, NOVOZYM™ FM 2.0L, and NOVOZYM™ 50006 (available from Novozymes A/S, Denmark) and GC106™ and SPEZYME™ FAN from Genencor Int., Inc., USA.

The protease or thermostable protease may be present in concentrations in the range from 0.0001 to 1.0 wt.-% of TS, preferably 0.001 to 0.1 wt.-% of TS.

In an embodiment the protease is a metalloprotease. In a preferred embodiment the protease is derived from a strain of the genus *Thermoascus,* preferably a strain of *Thermoascus aurantiacus,* especially *Thermoaccus aurantiacus* CGMCC No. 0670 having the sequence shown in the mature part of SEQ ID NO: 2 in WO 03/048353. The *Thermoaccus aurantiacus* protease is active from 20-90°C, with an optimum temperature around 70°C. Further, the enzyme has activity between pH 5-10 with an optimum around pH 6.

The protease used in a process of the invention, preferably added during liquefaction step i), may be a thermostable protease that has either:
i) a thermostability value of more than 20% determined as Relative Activity at 80°C/70°C; and/or
ii) a thermostability value of more than 10% determined as Relative Activity at 85°C/70°C.
In an embodiment the protease has a thermostability value:
- of more than 30%, more than 40%, more than 50%, more than 60%, more than 70%, more than 80%, more than 90% determined as Relative Activity at 80°C/70°C, and/or
- of more than 12%, more than 14%, more than 16%, more than 18%, more than 20%, determined as Relative Activity at 85°C/70°C; and/or
- of more than 20%, more than 30%, more than 40%, more than 50%, more than 60%, more than 70%, more than 80%, more than 90% determined as Remaining Activity at 80°C; and/or
of more than 20%, more than 30%, more than 40%, more than 50%, more than 60%, more than 70%, more than 80%, more than 90% determined as Remaining Activity at 84°C and/or.

Purified variants may have a themostability for above 90, above 100 at 85°C as determined using the Zein-BCA assay as disclosed in Example 3.

Determination of "Relative Activity" and "Remaining Activity" is determined as described in Example 2.

Proteases are classified on the basis of their catalytic mechanism into the following groups: Serine proteases (S), Cysteine proteases (C), Aspartic proteases (A), Metallo proteases (M), and Unknown, or as yet unclassified, proteases (U), see Handbook of Proteolytic Enzymes, A.J.Barrett, N.D.Rawlings, J.F.Woessner (eds), Academic Press (1998), in particular the general introduction part.

In a preferred embodiment the thermostable protease used in a process of the invention is a "metalloprotease" defined as a protease belonging to EC 3.4.24 (metalloendopeptidases); preferably EC 3.4.24.39 (acid metallo proteinases).

To determine whether a given protease is a metallo protease or not, reference is made to the above "Handbook of Proteolytic Enzymes" and the principles indicated therein. Such determination can be carried out for all types of proteases, be it naturally occurring or wild-type proteases; or genetically engineered or synthetic proteases.

Protease activity can be measured using any suitable assay, in which a substrate is employed, that includes peptide bonds relevant for the specificity of the protease in question. Assay-pH and assay-temperature are likewise to be adapted to the protease in question. Examples of assay-pH-values are pH 6, 7, 8, 9, 10, or 11. Examples of assay-temperatures are 30, 35, 37, 40, 45, 50, 55, 60, 65, 70 or 80°C.

Examples of protease substrates are casein, such as Azurine-Crosslinked Casein (AZCL-casein). Two protease assays are described below in the "Materials & Methods"-section, of which the so-called "AZCL-Casein Assay" is the preferred assay.

The thermostability protease may be a variant of, e.g., a wild-type protease as long as the protease has the thermostability properties defined above. In a preferred embodiment the protease is a variant of a metallo protease as defined above. In an embodiment the protease used in a process of the invention is of fungal origin, such as a fungal metallo protease, such as a fungal metallo protease derived from a strain of the genus *Thermoascus,* preferably a strain of *Thermoascus aurantiacus,* especially *Thermoascus aurantiacus* CGMCC No. 0670 (classified as EC 3.4.24.39).

In an embodiment the protease is a variant of the mature part of the metalloprotease shown in SEQ ID NO: 2 disclosed in WO 2003/048353 or the mature part of SEQ ID NO: 1 in WO 2010/008841 and shown as SEQ ID NO: 3 herein with the following mutations:
- S5*+D79L+S87P+A112P+D142L
- D79L+S87P+A112P+T124V+D142L
- S5*+N26R+D79L+S87P+A112P+D142L
- N26R+T46R+D79L+S87P+A112P+D142L
- T46R+D79L+S87P+T116V+D142L
- D79L+P81R+S87P+A112P+D142L
- A27K+D79L+S87P+A112P+T124V+D142L
- D79L+Y82F+S87P+A112P+T124V+D142L
- D79L+Y82F+S87P+A112P+T124V+D142L
- D79L+S87P+A112P+T124V+A126V+D142L
- D79L+S87P+A112P+D142L
- D79L+Y82F+S87P+A112P+D142L
- S38T+D79L+S87P+A112P+A126V+D142L
- D79L+Y82F+S87P+A112P+A126V+D142L
- A27K+D79L+S87P+A112P+A126V+D142L
- D79L+S87P+N98C+A112P+G135C+D142L
- D79L+S87P+A112P+D142L+T141C+M161C
- S36P+D79L+S87P+A112P+D142L
- A37P+D79L+S87P+A112P+D142L
- S49P+D79L+S87P+A112P+D142L
- S50P+D79L+S87P+A112P+D142L
- D79L+S87P+D104P+A112P+D142L
- D79L+Y82F+S87G+A112P+D142L
- S70V+D79L+Y82F+S87G+Y97W+A112P+D142L
- D79L+Y82F+S87G+Y97W+D104P+A112P+D142L
- S70V+D79L+Y82F+S87G+A112P+D142L
- D79L+Y82F+S87G+D104P+A112P+D142L
- D79L+Y82F+S87G+A112P+A126V+D142L
- Y82F+S87G+S70V+D79L+D104P+A112P+D142L
- Y82F+S87G+D79L+D104P+A112P+A126V+D142L
- A27K+D79L+Y82F+S87G+D104P+A112P+A126V+D142L
- A27K+Y82F+S87G+D104P+A112P+A126V+D142L
- A27K+D79L+Y82F+ D104P+A112P+A126V+D142L
- A27K+Y82F+D104P+A112P+A126V+D142L
- A27K+D79L+S87P+A112P+D142L.

### Glucoamylase Present and/or Added During Saccharification or SSF

According to the invention a glucoamylase having at least 80% identity to the mature polypeptide shown in SEQ ID NO: 9 is present and/or added during saccharification step ii) and/or fermentation step iii) such as SSF. Optionally a protease, such as a thermostable protease is also present or added. As mentioned above a pullulanase may also be present and/or added during saccharification step ii) and/or fermentation step iii) such as SSF.

In a preferred embodiment the glucoamylase is added together with or separately from the alpha-amylase and optionally the protease and/or pullulanase.

In an embodiment the glucoamylase has a heat stability at 85°C of at least 20%, at least 30%, preferably at least 35% relative activity. In an embodiment the glucoamylase has a relative activity at pH 4.5 of at least 80%, preferably at least 85%, preferably at least 90%, preferably at least 95%.

In a specific and preferred embodiment the glucoamylaseis derived from a strain of the genus *Penicillium,* especially a strain of *Penicillium oxalicum* disclosed as SEQ ID NO: 9 herein.

In an embodiment the thermostable glucoamylase used in a process of the invention has at least 80%, more preferably at least 85%, more preferably at least 90%, more preferably at least 91%, more preferably at least 92%, even more preferably at least 93%, most preferably at least 94%, and even most preferably at least 95%, such as even at least 96%, at least 97%, at least 98%, at least 99% or 100% identity to the mature polypeptide shown in SEQ ID NO: 2 in PCT/CN10/071753 or SEQ ID NO: 9 herein.

### Pullulanase Present and/or Added During A Process Step Of The Invention

Optionally a pullulanase (including amylopullulanases) may be present or added during liquefaction step i), saccharification step ii) and/or fermentation step iii), such as SSF. In an embodiment the pullulanse is a thermostable pullulanase added during liquefaction step i). A thermostable pullulanase may be present and/or added during liquefaction step i) together with an alpha-amylase and optionally a thermostable protease.

The pullulanase may be present and/or added during a process step of the invention, such as liquefaction step i), saccharification step ii), fermentation step iii) and/or simultaneous saccharification and fermentation (SSF).

Pullulanases (E.C. 3.2.1.41, pullulan 6-glucano-hydrolase), are debranching enzymes characterized by their ability to hydrolyze the alpha-1,6-glycosidic bonds in, for example, amylopectin and pullulan.

Contemplated pullulanases according to the present invention include the pullulanases from *Bacillus amyloderamificans* disclosed in U.S. Patent No. 4,560,651, the pullulanase disclosed as SEQ ID NO: 2 in WO 01/151620, the *Bacillus deramificans* disclosed as SEQ ID NO: 4 in WO 01/151620, and the pullulanase from *Bacillus acidopullulyticus* disclosed as SEQ ID NO: 6 in WO 01/151620 and also described in FEMS Mic. Let. (1994) 115, 97-106.

Additional pullulanases contemplated according to the present invention included the pullulanases from *Pyrococcus woesei,* specifically from *Pyrococcus woesei* DSM No. 3773 disclosed in WO 92/02614.

In an embodiment the pullulanase is a family GH57 pullulanase. In an embodiment the pullulanase includes an X47 domain as disclosed in PCT/US10/061761. More specifically the pullulanase may be derived from a strain of the genus *Thermococcus,* including *Thermococcus litoralis* and *Thermococcus hydrothermalis,* such as the *Thermococcus hydrothermalis* pullulanase shown in SEQ ID NO: 11 truncated at site X4 right after the X47 domain (i.e., amino acids 1-782 in SEQ ID NOS: 11 and 12). The pullulanase may also be a hybrid of the *Thermococcus litoralis* and *Thermococcus hydrothermalis* pullulanases or a T. *hydrothermalis*/*T. litoralis* hybrid enzyme with truncation site X4 disclosed in PCT/US10/061761 and disclosed in SEQ ID NO: 12.

The pullulanase may according to the invention be added in an effective amount which include the preferred amount of about 0.0001-10 mg enzyme protein per gram DS, preferably 0.0001-0.10 mg enzyme protein per gram DS, more preferably 0.0001-0.010 mg enzyme protein per gram DS. Pullulanase activity may be determined as NPUN. An Assay for determination of NPUN is described in the "Materials & Methods"-section below.

Suitable commercially available pullulanase products include PROMOZYME D, PROMOZYME™ D2 (Novozymes A/S, Denmark), OPTIMAX L-300 (Genencor Int., USA), and AMANO 8 (Amano, Japan).

Additional Carbohydrate-Source Generating Enzyme Present And/or Added During Saccharification and/or Fermentation

According to the invention an additional carbohydrate-source generating enzyme, preferably an additional glucoamylase, may be present and/or added during saccharification step ii) and/or fermentation step iii). The additional carbohydrate-source generating enzyme may also be an enzyme selected from the group consisting of: beta-amylase, maltogenic amylase and alpha-glucosidase.

In a preferred embodiment the additional carbohydrate-source generating enzyme is a glucoamylase of fungal origin, preferably from a stain of *Aspergillus,* preferably *A. niger, A. awamori,* or *A. oryzae;* or a strain of *Trichoderma,* preferably *T. reesei*; or a strain of *Talaromyces,* preferably *T. emersonii*,

Additional Glucoamylase Present and/or Added During Saccharification and/or Fermenation

According to the invention an additional glucoamylase may be present and/or added during saccharification step ii) and/or fermentation step iii), such as SSF. The additional glucoamylase may be derived from any suitable source, e.g., derived from a microorganism or a plant. Preferred additional glucoamylases are of fungal or bacterial origin, selected from the group consisting of *Aspergillus* glucoamylases, in particular *Aspergillus niger* G1 or G2 glucoamylase (Boel et al. (1984), EMBO J. 3 (5), p. 1097-1102), or variants thereof, such as those disclosed in WO 92/00381, WO 00/04136 and WO 01/04273 (from Novozymes, Denmark); the *A. awamori* glucoamylase disclosed in WO 84/02921, *Aspergillus oryzae* glucoamylase (Agric. Biol. Chem. (1991), 55 (4), p. 941-949), or variants or fragments thereof. Other *Aspergillus* glucoamylase variants include variants with enhanced thermal stability: G137A and G139A (Chen et al. (1996), Prot. Eng. 9, 499-505); D257E and D293E/Q (Chen et al. (1995), Prot. Eng. 8, 575-582); N182 (Chen et al. (1994), Biochem. J. 301, 275-281); disulphide bonds, A246C (Fierobe et al. (1996), Biochemistry, 35, 8698-8704; and introduction of Pro residues in position A435 and S436 (Li et al. (1997), Protein Eng. 10, 1199-1204.

Other additional glucoamylases include *Athelia rolfsii* (previously denoted *Corticium rolfsii*) glucoamylase (see US patent no. 4,727,026 and (Nagasaka et al. (1998) "Purification and properties of the raw-starch-degrading glucoamylases from Corticium rolfsii, Appl Microbiol Biotechnol 50:323-330), *Talaromyces* glucoamylases, in particular derived from *Talaromyces emersonii* (WO 99/28448), *Talaromyces leycettanus* (US patent no. Re. 32,153), *Talaromyces duponti, Talaromyces thermophilus* (US patent no. 4,587,215). In a preferred embodiment the glucoamylase used during saccharification and/or fermentation is the *Talaromyces emersonii* glucoamylase disclosed in WO 99/28448.

Additional bacterial glucoamylases contemplated include glucoamylases from the genus *Clostridium,* in particular *C*. *thermoamylolyticum* (EP 135,138), and *C*_{.} *thermohydrosulfuricum* (WO 86/01831) and *Trametes cingulata, Pachykytospora papyracea;* and *Leucopaxillus giganteus* all disclosed in WO 2006/069289; or *Peniophora rufomarginata* disclosed in WO2007/124285; or a mixture thereof. Also hybrid glucoamylase are contemplated according to the invention. Examples the hybrid glucoamylases disclosed in WO 2005/045018. Specific examples include the hybrid glucoamylase disclosed in Table 1 and 4 of Example 1.
In an embodiment the additional glucoamylase is derived from a strain of the genus *Pycnoporus*, in particular a strain of *Pycnoporus* as described in US 61/264,977, or from a strain of the genus *Gloephyllum*, in particular a strain of *Gloephyllum* as described in US 61/406,741 or a strain of the genus *Nigrofomes,* in particular a strain of *Nigrofomes sp.* disclosed in US 61/411,044.

Contemplated are also additional glucoamylases which exhibit a high identity to any of above mention glucoamylases, i.e., at least 70%, at least 75%, at least 80%, at least 85%, at least 90%, at least 95%, at least 96%, at least 97%, at least 98%, at least 99% or even 100% identity to the mature enzymes sequences mentioned above.

The additional glucoamylases may be added in an amount of 0.0001-20 AGU/g DS, preferably 0.001-10 AGU/g DS, especially between 0.01-5 AGU/g DS, such as 0.1-2 AGU/g DS.

Commercially available compositions comprising additional glucoamylase include AMG 200L; AMG 300 L; SAN™ SUPER, SAN™ EXTRA L, SPIRIZYME™ PLUS, SPIRIZYME™ FUEL, SPIRIZYME™ B4U, SPIRIZYME™ ULTRA and AMG™ E (from Novozymes A/S); OPTIDEX™ 300, GC480, GC417 (from Genencor Int.); AMIGASE™ and AMIGASE™ PLUS (from DSM); G-ZYME™ G900, G-ZYME™ and G990 ZR (from Genencor Int.).

### Maltogenic Amylase

The additional carbohydrate-source generating enzyme present and/or added during saccharification step ii) and/or fermentation step iii) may be a maltogenic alpha-amylase. A "maltogenic alpha-amylase" (glucan 1,4-alpha-maltohydrolase, E.C. 3.2.1.133) is able to hydrolyze amylose and amylopectin to maltose in the alpha-configuration. A maltogenic amylase from *Bacillus stearothermophilus* strain NCIB 11837 is commercially available from Novozymes A/S. Maltogenic alpha-amylases are described in US Patent nos. 4,598,048, 4,604,355 and 6,162,628.

The maltogenic amylase may in a preferred embodiment be added in an amount of 0.05-5 mg total protein/gram DS or 0.05-5 MANU/g DS.

### Materials & Methods

### Materials:

Alpha-Amylase A: *Bacillus stearothermophilus* alpha-amylase with the mutations I181*+G182*+N193F truncated to 491 amino acids (SEQ ID NO: 1 herein)
Glucoamylase T: *Talaromyces emersonii* glucoamylase disclosed in WO 99/28448 as SEQ ID NO: 7 and *Trametes cingulata* glucoamylase disclosed in WO 06/069289 in a ratio of about 9:1.
Yeast: RED STAR ETHANOL RED™ available from Red Star/Lesaffre, USA.

### Methods

**Identity:** The relatedness between two amino acid sequences or between two nucleotide sequences is described by the parameter "identity".

For purposes of the present invention the degree of identity between two amino acid sequences, as well as the degree of identity between two nucleotide sequences, may be determined by the program "align" which is a Needleman-Wunsch alignment (i.e. a global alignment). The program is used for alignment of polypeptide, as well as nucleotide sequences. The default scoring matrix BLOSUM50 is used for polypeptide alignments, and the default identity matrix is used for nucleotide alignments. The penalty for the first residue of a gap is -12 for polypeptides and -16 for nucleotides. The penalties for further residues of a gap are -2 for polypeptides, and -4 for nucleotides.

"Align" is part of the FASTA package version v20u6 (see W. R. Pearson and D. J. Lipman (1988), "Improved Tools for Biological Sequence Analysis", PNAS 85:2444-2448, and W. R. Pearson (1990) "Rapid and Sensitive Sequence Comparison with FASTP and FASTA," " Methods in Enzymology 183:63- 98). FASTA protein alignments use the Smith-Waterman algorithm with no limitation on gap size (see "Smith-Waterman algorithm", T. F. Smith and M. S. Waterman (1981) J. Mol. Biol. 147:195-197).

### Protease assays

### AZCL-casein assay

A solution of 0.2% of the blue substrate AZCL-casein is suspended in Borax/NaH₂PO₄ buffer pH9 while stirring. The solution is distributed while stirring to microtiter plate (100 microL to each well), 30 microL enzyme sample is added and the plates are incubated in an Eppendorf Thermomixer for 30 minutes at 45° C and 600 rpm. Denatured enzyme sample (100°C boiling for 20min) is used as a blank. After incubation the reaction is stopped by transferring the microtiter plate onto ice and the coloured solution is separated from the solid by centrifugation at 3000rpm for 5 minutes at 4° C. 60 microL of supernatant is transferred to a microtiter plate and the absorbance at 595nm is measured using a BioRad Microplate Reader.

### pNA-assay

50 microL protease-containing sample is added to a microtiter plate and the assay is started by adding 100 microL 1mM pNA substrate (5 mg dissolved in 100 microL DMSO and further diluted to 10 mL with Borax/NaH₂PO₄ buffer pH 9.0). The increase in OD₄₀₅ at room temperature is monitored as a measure of the protease activity.

### Glucoamylase activity (AGU)

Glucoamylase activity may be measured in Glucoamylase Units (AGU).

The Novo Glucoamylase Unit (AGU) is defined as the amount of enzyme, which hydrolyzes 1 micromole maltose per minute under the standard conditions 37°C, pH 4.3, substrate: maltose 23.2 mM, buffer: acetate 0.1 M, reaction time 5 minutes.

An autoanalyzer system may be used. Mutarotase is added to the glucose dehydrogenase reagent so that any alpha-D-glucose present is turned into beta-D-glucose. Glucose dehydrogenase reacts specifically with beta-D-glucose in the reaction mentioned above, forming NADH which is determined using a photometer at 340 nm as a measure of the original glucose concentration.

| AMG incubation: | |
|---|---|
| Substrate: | maltose 23.2 mM |
| Buffer: | acetate 0.1 M |
| pH: | 4.30 ± 0.05 |
| Incubation temperature: | 37°C ± 1 |
| Reaction time: | 5 minutes |
| Enzyme working range: | 0.5-4.0 AGU/mL |

| Color reaction: | |
|---|---|
| GlucDH: | 430 U/L |
| Mutarotase: | 9 U/L |
| NAD: | 0.21 mM |
| Buffer: | phosphate 0.12 M; 0.15 M NaCl |
| pH: | 7.60 ± 0.05 |
| Incubation temperature: | 37°C ± 1 |
| Reaction time: | 5 minutes |
| Wavelength: | 340 nm |

A folder (EB-SM-0131.02/01) describing this analytical method in more detail is available on request from Novozymes A/S, Denmark, which folder is hereby included by reference.

### Alpha-Amylase activity (KNU)

Alpha-amylase activity may be determined using potato starch as substrate. This method is based on the break-down of modified potato starch by the enzyme, and the reaction is followed by mixing samples of the starch/enzyme solution with an iodine solution. Initially, a blackish-blue color is formed, but during the break-down of the starch the blue color gets weaker and gradually turns into a reddish-brown, which is compared to a colored glass standard.

One Kilo Novo alpha amylase Unit (KNU) is defined as the amount of enzyme which, under standard conditions (i.e., at 37°C +/- 0.05; 0.0003 M Ca²⁺; and pH 5.6) dextrinizes 5260 mg starch dry substance Merck Amylum solubile.

A folder EB-SM-0009.02/01 describing this analytical method in more detail is available upon request to Novozymes A/S, Denmark, which folder is hereby included by reference.

### KNU(S) Activity

KNU(S) is used to determine the activity of *Bacillus stearothermophilus* and is described on **Determination of pullulanase activity (NPUN)**
Endo-pullulanase activity in NPUN is measured relative to a Novozymes pullulanase standard. One pullulanase unit (NPUN) is defined as the amount of enzyme that releases 1 micro mol glucose per minute under the standard conditions (0.7% red pullulan (Megazyme), pH 5, 40°C, 20 minutes). The activity is measured in NPUN/ml using red pullulan.

1 mL diluted sample or standard is incubated at 40°C for 2 minutes. 0.5 mL 2% red pullulan, 0.5 M KCI, 50 mM citric acid, pH 5 are added and mixed. The tubes are incubated at 40°C for 20 minutes and stopped by adding 2.5 ml 80% ethanol. The tubes are left standing at room temperature for 10-60 minutes followed by centrifugation 10 minutes at 4000 rpm. OD of the supernatants is then measured at 510 nm and the activity calculated using a standard curve.

### Determination of Maltogenic Amylase activity (MANU)

One MANU (Maltogenic Amylase Novo Unit) may be defined as the amount of enzyme required to release one micro mole of maltose per minute at a concentration of 10 mg of maltotriose (Sigma M 8378) substrate per ml of 0.1 M citrate buffer, pH 5.0 at 37°C for 30 minutes.

### EXAMPLES

### Example 1

### Stability of Alpha-Amylase Variants

The stability of a reference alpha-amylase (*Bacillus stearothermophilus* alpha-amylase with the mutations I181*+G182*+N193F truncated to 491 amino acids (SEQ ID NO: 1)) and alpha-amylase variants thereof was determined by incubating the reference alpha-amylase and variants at pH 4.5 and 5.5 and temperatures of 75°C and 85°C with 0.12 mM CaCl₂ followed by residual activity determination using the EnzChek® substrate (EnzChek® Ultra Amylase assay kit, E33651, Molecular Probes).

Purified enzyme samples were diluted to working concentrations of 0.5 and 1 or 5 and 10 ppm (micrograms/ml) in enzyme dilution buffer (10 mM acetate, 0.01% Triton X100, 0.12 mM CaCl₂, pH 5.0). Twenty microliters enzyme sample was transferred to 48-well PCR MTP and 180 microliters stability buffer (150 mM acetate, 150 mM MES, 0.01% Triton X100, 0.12 mM CaCl₂, pH 4.5 or 5.5) was added to each well and mixed. The assay was performed using two concentrations of enzyme in duplicates. Before incubation at 75°C or 85°C, 20 microliters was withdrawn and stored on ice as control samples. Incubation was performed in a PCR machine at 75°C and 85°C.After incubation samples were diluted to 15 ng/mL in residual activity buffer (100 mM Acetate, 0.01% Triton X100, 0.12 mM CaCl₂, pH 5.5) and 25 microliters diluted enzyme was transferred to black 384-MTP. Residual activity was determined using the EnzChek substrate by adding 25 microliters substrate solution (100 micrograms/ml) to each well. Fluorescence was determined every minute for 15 minutes using excitation filter at 485-P nm and emission filter at 555 nm (fluorescence reader is Polarstar, BMG). The residual activity was normalized to control samples for each setup.
Assuming logarithmic decay half life time (T½ (min)) was calculated using the equation: T½ (min) = T(min)*LN(0.5)/LN(%RA/100), where T is assay incubation time in minutes, and %RA is % residual activity determined in assay.

Using this assay setup the half life time was determined for the reference alpha-amylase and variant thereof as shown in Table 1.

**Table 1**

| **Mutations** | **T½ (min) (pH 4.5, 75°C, 0.12 mM CaCl₂)** | **T½ (min) (pH 4.5, 85°C, 0.12 mM CaCl₂)** | **T½ (min) (pH 5.5, 85°C, 0.12 mM CaCl₂)** |
|---|---|---|---|
| Reference Alpha-Amylase A | 21 | 4 | 111 |
| Reference Alpha-Amylase A with the substitution V59A | 32 | 6 | 301 |
| Reference Alpha-Amylase A with the substitution V59E | 28 | 5 | 230 |
| Reference Alpha-Amylase A with the substitution V59I | 28 | 5 | 210 |
| Reference Alpha-Amylase A with the substitution V59Q | 30 | 6 | 250 |
| Reference Alpha-Amylase A with the substitutions V59A+Q89R+ G112D+E129V+K177L+R179E+ K220P+N224L+Q254S | 149 | 22 | ND |
| Reference Alpha-Amylase A with the substitutions V59A+Q89R+E129V+K177L+R179E+H208Y+K220P+ N224L+Q254S | >180 | 28 | ND |
| Reference Alpha-Amylase A with the substitutions V59A+Q89R+E129V+ K177L+R179E+K220P+N224L+ Q254S+D269E+D281N | 112 | 16 | ND |
| Reference Alpha-Amylase A with the substitutions V59A+Q89R+E129V+ K177L+R179E+K220P+N224L+ Q254S+1270L | 168 | 21 | ND |
| Reference Alpha-Amylase A with the substitutions V59A+Q89R+E129V+ K177L+R179E+K220P+N224L+ Q254S+H274K | >180 | 24 | ND |
| Reference Alpha-Amylase A with the substitutions V59A+Q89R+E129V+ K177L+R179E+K220P+N224L+ Q254S+Y276F | 91 | 15 | ND |
| Reference Alpha-Amylase A with the substitutions V59A+E129V+ R157Y+K177L+R179E+K220P+ N224L+S242Q+Q254S | 141 | 41 | ND |
| Reference Alpha-Amylase A with the substitutions V59A+E129V+ K177L+R179E+H208Y+K220P+ N224L+S242Q+Q254S | >180 | 62 | ND |
| Reference Alpha-Amylase A with the substitutions V59A+E129V+ K177L+R179E+K220P+N224L+ S242Q+Q254S | >180 | 49 | >480 |
| Reference Alpha-Amylase A with the substitutions V59A+E129V+ K177L+R179E+K220P+N224L+ S242Q+Q254S+H274K | >180 | 53 | ND |
| Reference Alpha-Amylase A with the substitutions V59A+E129V+ K177L+R179E+K220P+N224L+ S242Q+Q254S+Y276F | >180 | 57 | ND |
| Reference Alpha-Amylase A with the substitutions V59A+E129V+ K177L+R179E+K220P+N224L+ S242Q+Q254S+D281N | >180 | 37 | ND |
| Reference Alpha-Amylase A with the substitutions V59A+E129V+ K177L+R179E+K220P+N224L+ S242Q+Q254S+M284T | >180 | 51 | ND |
| Reference Alpha-Amylase A with the substitutions V59A+E129V+ K177L+R179E+K220P+N224L+ S242Q+Q254S+G416V | >180 | 45 | ND |
| Reference Alpha-Amylase A with the substitutions V59A+E129V+ K177L+R179E+K220P+N224L+ Q254S | 143 | 21 | >480 |
| Reference Alpha-Amylase A with the substitutions V59A+E129V+ K177L+R179E+K220P+N224L+ Q254S+M284T | >180 | 22 | ND |
| Reference Alpha-Amylase A with the substitutions A91L+M961+E129V+ K177L+R179E+K220P+N224L+ S242Q+Q254S | >180 | 38 | ND |
| Reference Alpha-Amylase A with the substitutions E129V+K177L+ R179E | 57 | 11 | 402 |
| Reference Alpha-Amylase A with the substitutions E129V+K177L+ R179E+K220P+N224L+S242Q+ Q254S | 174 | 44 | >480 |
| Reference Alpha-Amylase A with the substitutions E129V+K177L+R179E+K220P+N224L+S242Q+ Q254S+Y276F+L427M | >180 | 49 | >480 |
| Reference Alpha-Amylase A with the substitutions E129V+K177L+ R179E+K220P+N224L+S242Q+ Q254S+M284T | >180 | 49 | >480 |
| Reference Alpha-Amylase A with the substitutions E129V+K177L+ R179E+K220P+N224L+S242Q+ Q254S+N376*+I377* | 177 | 36 | >480 |
| Reference Alpha-Amylase A with the substitutions E129V+K177L+ R179E+K220P+N224L+Q254S | 94 | 13 | >480 |
| Reference Alpha-Amylase A with the substitutions E129V+K177L+ R179E+K220P+N224L+Q254S+ M284T | 129 | 24 | >480 |
| Reference Alpha-Amylase A with the substitutions E129V+K177L+ R179E+S242Q | 148 | 30 | >480 |
| Reference Alpha-Amylase A with the substitutions E129V+K177L+ R179V | 78 | 9 | >480 |
| Reference Alpha-Amylase A with the substitutions E129V+K177L+R179V+K220P+N224L+S242Q+ Q254S | 178 | 31 | >480 |
| Reference Alpha-Amylase A with the substitutions K220P+N224L+ S242Q+Q254S | 66 | 17 | >480 |
| Reference Alpha-Amylase A with the substitutions K220P+N224L+ Q254S | 30 | 6 | 159 |
| Reference Alpha-Amylase A with the substitution M284T | 35 | 7 | 278 |
| Reference Alpha-Amylase A with the substitutions M284V | 59 | 13 | ND |

| | | | |
|---|---|---|---|
| ND not determined | | | |

The results demonstrate that the alpha-amylase variants have a significantly greater half-life and stability than the reference alpha-amylase.

### Example 2

### Preparation of Protease Variants and Test of Thermostability

Chemicals used were commercial products of at least reagent grade.

### Strains and plasmids:

*E.coli* DH12S (available from Gibco BRL) was used for yeast plasmid rescue. pJTP000 is a S. *cerevisiae* and *E.coli* shuttle vector under the control of TPI promoter, constructed from pJC039 described in WO 01/92502, in which the *Thermoascus aurantiacus* M35 protease gene (WO 03048353) has been inserted.

*Saccharomyces cerevisiae* YNG318 competent cells: MATa Dpep4[cir+] ura3-52, leu2-D2, his 4-539 was used for protease variants expression. It is described in J. Biol. Chem. 272 (15), pp 9720-9727, 1997.

### Media and substrates

10X Basal solution: Yeast nitrogen base w/o amino acids (DIFCO) 66.8 g/L, succinate 100 g/l, NaOH 60 g/l.
SC-glucose: 20 % glucose (i.e., a final concentration of 2 % = 2 g/100 mL)) 100 mL/L, 5 % threonine 4 mL/L, 1 % tryptophan10 ml/l, 20 % casamino acids 25 ml/l, 10 X basal solution 100 ml/l. The solution is sterilized using a filter of a pore size of 0.20 micrometer. Agar (2%) and H₂O (approx. 761 mL) is autoclaved together, and the separately sterilized SC-glucose solution is added to the agar solution.
YPD: Bacto peptone 20 g/l, yeast extract 10 g/L, 20 % glucose 100 mL/L.
YPD+Zn: YPD+0.25 mM ZnSO₄.
PEG/LiAc solution: 40 % PEG4000 50 ml, 5 M Lithium Acetate 1 mL.
96 well Zein micro titre plate:
Each well contains 200 microL of 0.05-0.1 % of zein (Sigma), 0.25 mM ZnSO₄ and 1 % of agar in 20 mM sodium acetate buffer, pH 4.5.

### DNA manipulations

Unless otherwise stated, DNA manipulations and transformations were performed using standard methods of molecular biology as described in Sambrook et al. (1989) Molecular cloning: A laboratory manual, Cold Spring Harbor lab. Cold Spring Harbor, NY; Ausubel, F. M. et al. (eds.) "Current protocols in Molecular Biology", John Wiley and Sons, 1995; Harwood, C. R. and Cutting, S. M. (Eds.).

### Yeast transformation

Yeast transformation was performed using the lithium acetate method. 0.5 microL of vector (digested by restriction endnucleases) and 1 microL of PCR fragments is mixed. The DNA mixture, 100 microL of YNG318 competent cells, and 10 microL of YEAST MAKER carrier DNA (Clontech) is added to a 12 mL polypropylene tube (Falcon 2059). Add 0.6 mL PEG/LiAc solution and mix gently. Incubate for 30 min at 30°C, and 200 rpm followed by 30 min at 42°C (heat shock). Transfer to an eppendorf tube and centrifuge for 5 sec. Remove the supernatant and resolve in 3 mL of YPD. Incubate the cell suspension for 45 min at 200 rpm at 30°C. Pour the suspension to SC-glucose plates and incubate 30°C for 3 days to grow colonies. Yeast total DNA are extracted by Zymoprep Yeast Plasmid Miniprep Kit (ZYMO research).

### DNA sequencing

*E.coli* transformation for DNA sequencing was carried out by electroporation (BIO-RAD Gene Pulser). DNA Plasmids were prepared by alkaline method (Molecular Cloning, Cold Spring Harbor) or with the Qiagen® Plasmid Kit. DNA fragments were recovered from agarose gel by the Qiagen gel extraction Kit. PCR was performed using a PTC-200 DNA Engine. The ABI PRISMTM 310 Genetic Analyzer was used for determination of all DNA sequences.

### Construction of protease expression vector

The *Themoascus* M35 protease gene was amplified with the primer pair Prot F (SEQ ID NO: 4) and Prot R (SEQ ID NO: 5). The resulting PCR fragments were introduced into *S*. *cerevisiae* YNG318 together with the pJC039 vector (described in WO 2001/92502) digested with restriction enzymes to remove the Humicola insolens cutinase gene.

The Plasmid in yeast clones on SC-glucose plates was recovered to confirm the internal sequence and termed as pJTP001.

### Construction of yeast library and site-directed variants

Library in yeast and site-directed variants were constructed by SOE PCR method (Splicing by Overlap Extension, see "PCR: A practical approach", p. 207-209, Oxford University press, eds. McPherson, Quirke, Taylor), followed by yeast *in vivo* recombination.

### General primers for amplification and sequencing

The primers AM34 (SEQ ID NO: 6) and AM35 (SEQ ID NO:7) were used to make DNA fragments containing any mutated fragments by the SOE method together with degenerated primers (AM34 + Reverse primer and AM35 + forward primer) or just to amplify a whole protease gene (AM34 + AM35).

| PCR reaction system: | Conditions: | |
|---|---|---|
| 48.5 microL H₂O | 1 | 94 °C 2 min |
| 2 beads puRe Taq Ready-To-Go PCR (Amersham Biosciences) | 2 | 94 °C 30 sec |
| 0.5 microL X 2 100 pmole/microL of primers | 3 | 55 °C 30 sec |
| 0.5 microL template DNA | 4 | 72 °C 90 sec |
| | 2-4 | 25 cycles |
| | 5 | 72 °C 10 min |

DNA fragments were recovered from agarose gel by the Qiagen gel extraction Kit. The resulting purified fragments were mixed with the vector digest. The mixed solution was introduced into *Saccharomyces cerevisiae* to construct libraries or site-directed variants by *in vivo* recombination.

### Relative activity assay

Yeast clones on SC-glucose were inoculated to a well of a 96-well micro titre plate containing YPD+Zn medium and cultivated at 28°C for 3 days. The culture supernatants were applied to a 96-well zein micro titer plate and incubated at at least 2 temperatures (ex., 70 °C and 80°C) for more than 4 hours or overnight. The turbidity of zein in the plate was measured as A630 and the relative activity (higher/lower temperatures) was determined as an indicator of thermoactivity improvement. The clones with higher relative activity than the parental variant were selected and the sequence was determined.

### Remaining activity assay

Yeast clones on SC-glucose were inoculated to a well of a 96-well micro titre plate and cultivated at 28 °C for 3 days. Protease activity was measured at 65°C using azo-casein (Megazyme) after incubating the culture supernatant in 20 mM sodium acetate buffer, pH 4.5, for 10 min at a certain temperature (80°C or 84°C with 4°C as a reference) to determine the remaining activity. The clones with higher remaining activity than the parental variant were selected and the sequence was determined.

### Azo-casein assay

20 microL of samples were mixed with 150 microL of substrate solution (4 mL of 12.5% azo-casein in ethanol in 96 mL of 20 mM sodium acetate, pH 4.5, containing 0.01 % triton-100 and 0.25 mM ZnSO₄) and incubated for 4 hours or longer.

After adding 20 microL/well of 100 % trichloroacetic acid (TCA) solution, the plate was centrifuge and 100 microL of supernatants were pipette out to measure A440.

### Expression of protease variants in Aspergillus oryzae

The constructs comprising the protease variant genes were used to construct expression vectors for *Aspergillus.* The *Aspergillus* expression vectors consist of an expression cassette based on the *Aspergillus niger* neutral amylase II promoter fused to the *Aspergillus nidulans* triose phosphate isomerase non translated leader sequence (Pna2/tpi) and the *Aspergillus niger* amyloglycosidase terminator (Tamg). Also present on the plasmid was the *Aspergillus selective* marker amdS from *Aspergillus nidulans* enabling growth on acetamide as sole nitrogen source. The expression plasmids for protease variants were transformed into *Aspergillus* as described in Lassen et al. (2001), Appl. Environ. Microbiol. 67, 4701-4707. For each of the constructs 10-20 strains were isolated, purified and cultivated in shake flasks.

### Purification of expressed variants

1. Adjust pH of the 0.22 µm filtered fermentation sample to 4.0.
2. Put the sample on an ice bath with magnetic stirring. Add (NH₄)₂SO₄ in small aliquots (corresponding to approx. 2.0-2.2 M (NH4)2SO4 not taking the volume increase into account when adding the compound).
3. After the final addition of (NH₄)₂SO₄, incubate the sample on the ice bath with gentle magnetic stirring for min. 45 min.
4. Centrifugation: Hitachi himac CR20G High-Speed Refrigerated Centrifuge equipped with R20A2 rotor head, 5°C, 20,000 rpm, 30 min.
5. Dissolve the formed precipitate in 200 mL 50 mM Na-acetate pH 4.0.
6. Filter the sample by vacuum suction using a 0.22 micro m PES PLUS membrane (IWAKI).
7. Desalt/buffer-exchange the sample to 50 mM Na-acetate pH 4.0 using ultrafiltration (Vivacell 250 from Vivascience equipped with 5 kDa MWCO PES membrane) overnight in a cold room. Dilute the retentate sample to 200 ml using 50 mM Na-acetate pH 4.0. The conductivity of sample is preferably less than 5 mS/cm.
8. Load the sample onto a cation-exchange column equilibrated with 50 mM Na-acetate pH 4.0. Wash unbound sample out of the column using 3 column volumes of binding buffer (50 mM Na-acetate pH 4.0), and elute the sample using a linear gradient, 0-100% elution buffer (50 mM Na-acetate + 1 M NaCl pH 4.0) in 10 column volumes.
9. The collected fractions are assayed by an endo-protease assay (cf. below) followed by standard SDS-PAGE (reducing conditions) on selected fractions. Fractions are pooled based on the endo-protease assay and SDS-PAGE.

### Endo-protease assay

1. Protazyme OL tablet/5 ml 250 mM Na-acetate pH 5.0 is dissolved by magnetic stirring (substrate: endo-protease Protazyme AK tablet from Megazyme - cat. # PRAK 11/08).
2. With stirring, 250 microL of substrate solution is transferred to a 1.5 mL Eppendorf tube.
3. 25 microL of sample is added to each tube (blank is sample buffer).
4. The tubes are incubated on a Thermomixer with shaking (1000 rpm) at 50°C for 15 minutes.
5. 250 microL of 1 M NaOH is added to each tube, followed by vortexing.
6. Centrifugation for 3 min. at 16,100 × G and 25°C.
7. 200 microL of the supernatant is transferred to a MTP, and the absorbance at 590 nm is recorded.

| Table 2. Relative Activity of protease variants. Numbering of substitution(s) starts from N-terminal of the mature peptide in amino acids 1 to 177 of SEQ ID NO: 3. | | | |
|---|---|---|---|
| **Variant** | **Substitution(s) and/or deletion(s)** | **Remaining Activity** | |
| | | **80°C** | **84°C** |
| JTP082 | ΔS5/D79L/S87P/A112P/D142L | | 53% |
| JTP091 | D79L/S87P/A112P/T124V/D142L | 43% | |
| JTP092 | ΔS5/N26R/D79L/S87P/A112P/D142L | | 60% |
| JTP095 | N26R/T46R/D79L/S87P/A112P/D142L | | 62% |
| JTP096 | T46R/D79L/S87P/T116V/D142L | | 67% |
| JTP099 | D79L/P81R/S87P/A112P/D142L | | 80% |
| JTP101 | A27K/D79L/S87P/A112P/T124V/D142L | 81% | |
| JTP116 | D79L/Y82F/S87P/A112P/T124V/D142L | 59% | |
| JTP117 | D79L/Y82F/S87P/A112P/T124V/D142L | 94% | |
| JTP127 | D79L/S87P/A112P/T124V/A126V/D142L | 53% | |

| Table 3 Relative Activity of protease variants. Numbering of substitution(s) starts from N-terminal of the mature peptide in amino acids 1 to 177 of SEQ ID NO: 3. | | | |
|---|---|---|---|
| **Variant** | **Substitutions** | **Relative Activity** | |
| | | **80°C/70°C** | **85°C/70°C** |
| JTP050 | D79L S87P A112P D142L | 23% | 9% |
| JTP134 | D79L Y82F S87P A112P D142L | 40% | |
| JTP135 | S38T D79L S87P A112P A126V D142L | 62% | |
| JTP136 | D79L Y82F S87P A112P A126V D142L | 59% | |
| JTP137 | A27K D79L S87P A112P A126V D142L | 54% | |
| JTP145 | S49P D79L S87P A112P D142L | 59% | |
| JTP146 | S50P D79L S87P A112P D142L | 63% | |
| JTP148 | D79L S87P D104P A112P D142L | 64% | |
| JTP161 | D79L Y82F S87G A112P D142L | 30% | 12% |
| JTP180 | S70V D79L Y82F S87G Y97W A112P D142L | 52% | |
| JTP181 | D79L Y82F S87G Y97W D104P A112P D142L | 45% | |
| JTP187 | S70V D79L Y82F S87G A112P D142L | 45% | |
| JTP188 | D79L Y82F S87G D104P A112P D142L | 43% | |
| JTP189 | D79L Y82F S87G A112P A126V D142L | 46% | |
| JTP193 | Y82F S87G S70V D79L D104P A112P D142L | | 15% |
| JTP194 | Y82F S87G D79L D104P A112P A126V D142L | | 22% |
| JTP196 | A27K D79L Y82F S87G D104P A112P A126V D142L | | 18% |

| Table 4 Relative Activity of protease variants. Numbering of substitution(s) starts from N-terminal of the mature peptide in amino acids 1 to 177 of SEQ ID NO: 3. | | | |
|---|---|---|---|
| | | | **Relative Activity** |
| **Variant** | **Substitutions** | | **80°C/70°C** |
| JTP196 | A27K D79L Y82F S87G D104P A112P A126V D142L | | 55% |
| JTP210 | A27K Y82F S87G D104P A112P A126V D142L | | 36% |
| JTP211 | A27K D79L Y82F D104P A112P A126V D142L | | 44% |
| JTP213 | A27K Y82F D104P A112P A126V D142L | | 37% |

### Example 3.

### Temperature Profile of Selected Protease Variants Using Purified Enzymes

Selected protease variants showing good thermostability were purified and the purified enzymes were used in a zein-BCA assay as described below. The remaining protease activity was determined at 60°C after incubation of the enzyme at elevated temperatures as indicated for 60 min.

### Zein-BCA assay:

Zein-BCA assay was performed to detect soluble protein quantification released from zein by variant proteases at various temperatures.

### Protocol:

1) Mix 10 microL of 10 micro g/mL enzyme solutions and 100 microL of 0.025% zein solution in a micro titer plate (MTP).
2) Incubate at various temperatures for 60 min.
3) Add 10 microL of 100% trichloroacetic acid (TCA) solution.
4) Centrifuge MTP at 3500rpm for 5 min.
5) Take out 15 microL to a new MTP containing 100 microL of BCA assay solution (Pierce Cat#:23225, BCA Protein Assay Kit).
6) Incubate for 30 min. at 60°C.
7) Measure A562.

The results are shown in Table 5. All of the tested protease variants showed an improved thermostability as compared to the wild type (WT) protease.

**Table 5 Zein-BCA assay**

| WT/Variant | Sample incubated 60 min at indicated temperatures (°C) | | | | | | |
|---|---|---|---|---|---|---|---|
| | (micro g/mL Bovine serum albumin equivalent peptide released) | | | | | | |
| | 60°C | 70°C | 75°C | 80°C | 85°C | 90°C | 95°C |
| WT | 94 | 103 | 107 | 93 | 58 | 38 | |
| JTP050 | 86 | 101 | 107 | 107 | 104 | 63 | 36 |
| (D79L+S87P+A112P+D142L) | | | | | | | |
| JTP077 | 82 | 94 | 104 | 105 | 99 | 56 | 31 |
| (A27K+D79L+S87P+A112P+D142L) | | | | | | | |
| JTP188 | 71 | 83 | 86 | 93 | 100 | 75 | 53 |
| (D79L+Y82F+S87G+D104P+A112P+D1 42L) | | | | | | | |
| JTP196 | 87 | 99 | 103 | 106 | 117 | 90 | 38 |
| (A27K+D79L+Y82F+S87G+D104P+A11 2P+A126V+D142L) | | | | | | | |

### Example 4

### Characterization of Penicillium oxalicum glucoamylase

The *Penicillium oxalicum* glucoamylase is disclosed in SEQ ID NO: 9 herein.
**Substrate.** Substrate: 1% soluble starch (Sigma S-9765) in deionized water
Reaction buffer: 0.1 M Acetate buffer at pH 5.3
Glucose concentration determination kit: Wako glucose assay kit (LabAssay glucose, WAKO, Cat# 298-65701).

**Reaction condition.** 20 microL soluble starch and 50 microL acetate buffer at pH5.3 were mixed. 30 microL enzyme solution (50 micro g enzyme protein/ml) was added to a final volume of 100 microL followed by incubation at 37°C for 15 min.

The glucose concentration was determined by Wako kits.

All the work carried out in parallel.

**Temperature optimum.** To assess the temperature optimum of the *Penicillium oxalicum* glucoamylase the "Reaction condition"-assay described above was performed at 20, 30, 40, 50, 60, 70, 80, 85, 90 and 95°C. The results are shown in Table 6.

**Table 6 Temperature optimum**

| | | | | | | | | | | |
|---|---|---|---|---|---|---|---|---|---|---|
| Temperature (°C) | 20 | 30 | 40 | 50 | 60 | 70 | 80 | 85 | 90 | 95 |
| Relative activity (%) | 63.6 | 71.7 | 86.4 | 99.4 | 94.6 | 100.0 | 92.9 | 92.5 | 82.7 | 82.8 |

From the results it can be seen that the optimal temperature for *Penicillium oxalicum* glucoamylase at the given conditions is between 50°C and 70°C and the glucoamylase maintains more than 80% activity at 95°C.

**Heat stability.** To assess the heat stability of the *Penicillium oxalicum* glucoamylase the Reaction condition assay was modifed in that the the enzyme solution and acetate buffer was preincubated for 15 min at 20, 30, 40, 50, 60, 70, 75, 80, 85, 90 and 95°C. Following the incubation 20 microL of starch was added to the solution and the assay was performed as described above.

The results are shown in Table 7.

**Table 7 Heat stability**

| | | | | | | | | | | |
|---|---|---|---|---|---|---|---|---|---|---|
| Temperature (°C) | 20 | 30 | 40 | 50 | 60 | 70 | 80 | 85 | 90 | 95 |
| Relative activity (%) | 91.0 | 92.9 | 88.1 | 100.0 | 96.9 | 86.0 | 34.8 | 36.0 | 34.2 | 34.8 |

From the results it can be seen that *Penicillium oxalicum* glucoamylase is stable up to 70 °C after preincubation for 15 min in that it maintains more than 80% activity.

**pH optimum.** To assess the pH optimum of the *Penicillium oxalicum* glucoamylase the Reaction condition assay described above was performed at pH 2.0, 3.0, 3.5, 4.0, 4.5, 5.0, 6.0 7.0, 8.0, 9.0, 10.0 and 11.0. Instead of using the acetate buffer described in the Reaction condition assay the following buffer was used 100mM Succinic acid, HEPES, CHES, CAPSO, 1mM CaCl₂, 150mM KCI, 0.01% Triton X-100, pH adjusted to 2.0, 3.0, 3.5, 4.0, 4.5, 5.0, 6.0 7.0, 8.0, 9.0, 10.0 or 11.0 with HCl or NaOH.

The results are shown in Table 8.

**Table 8 pH optimum**

| | | | | | | | | | | | | |
|---|---|---|---|---|---|---|---|---|---|---|---|---|
| pH | 2.0 | 3.0 | 3.5 | 4.0 | 4.5 | 5.0 | 6.0 | 7.0 | 8.0 | 9.0 | 10.0 | 11.0 |
| Relative activity (%) | 71.4 | 78.6 | 77.0 | 91.2 | 84.2 | 100.0 | 55.5 | 66.7 | 30.9 | 17.8 | 15.9 | 16.1 |

From the results it can be seen that *Penicillium oxalicum* glucoamylase at the given conditions has the highest activity at pH 5.0. The *Penicillium oxalicum* glucoamylase is active in a broad pH range in the it maintains more than 50% activity from pH 2 to 7.

**pH stability.** To assess the heat stability of the *Penicillium oxalicum* glucoamylase the Reaction condition assay was modifed in that the enzyme solution (50 micro g/mL) was pre-incubated for 20 hours in buffers with pH 2.0, 3.0, 3.5, 4.0, 4.5, 5.0, 6.0 7.0, 8.0, 9.0, 10.0 and 11.0 using the buffers described under pH optimum. After pre-incubation, 20 microL soluble starch to a final volume of 100 microL was added to the solution and the assay was performed as described above.

The results are shown in Table 9.

**Table 9 pH stability**

| | | | | | | | | | | | | |
|---|---|---|---|---|---|---|---|---|---|---|---|---|
| pH | 2.0 | 3.0 | 3.5 | 4.0 | 4.5 | 5.0 | 6.0 | 7.0 | 8.0 | 9.0 | 10.0 | 11.0 |
| Relative activity (%) | 17.4 | 98.0 | 98.0 | 103.2 | 100.0 | 93.4 | 71.2 | 90.7 | 58.7 | 17.4 | 17.0 | 17.2 |

From the results it can be seen that *Penicillium oxalicum* glucoamylase, is stable from pH 3 to pH 7 after preincubation for 20 hours and it decreases its activity at pH 8.

### Example 5: Evaluation of the Penicillium oxalicum glucoamylase in pre-saccharification at pH 5.0 and 70°C

This example describes an ethanol production process where thermostable *Penicillium oxalicum* glucoamylase is used in a pre-saccharification process followed by a fermentation process without using additional glucoamylase. This process is compared with a traditional ethanol production process where the liquefication process is followed by a SSF process using a non-heatstable glucoamylase.

**Slurry Sample.** A slurry sample prepared in the pilot plant at the National Corn to Ethanol Research Center (NCERC) during May of 2008 was used as a starting material for this study. The sample was prepared using whole ground corn (no backset) at a target % dry solids of 32.0%. It was liquefied at pH 5.80, 85°C for 30 minutes using 0.0246 KNU-s/g DS of Alpha-Amylase A.

**Liquefaction Treatment.** One fully-liquefied corn mash was prepared using the NCERC slurry sample described above as starting material. Briefly, approximately 400g of the slurry mash was weighed into a 1 L Nalgene bottle; the slurry mash sample was adjusted to pH 5.80 using 40% H₂SO₄. 0.0740 KNU-s/g DS of Alpha-Amylase A was added to the mash which was then incubated at 85°C for an additional 1.5 hours in a preheated water bath, with periodic mixing.

**pre-saccharification.** After incubation, the mash was aliquoted into two 100mL Nalgene bottles, cooled down to 70°C in a water bath and aliquots of *Penicillium oxalicum* glucoamylase were added to each mash to reach final enzyme of 0 and 0.24 AGU/gDS. The mashes were incubated at 70°C for an additional 2 hours in the water bath, with periodic mixing. After incubation, the mashes were immediately cooled to room temperature. The mashes were adjusted to pH 5.0 using 10% NaOH and urea and penicillin were added to reach final concentrations of 1000 ppm and 3 ppm, respectively. Small samples of each mash were taken for HPLC analysis.

The results are shown in table 10. The results show that the percentage of starch converted into glucose in pre-saccharification was enhanced by the addition of *Penicillium oxalicum* glucoamylase.

**Table 10**

| **Mash** | **Glucose (g/L)** | **% Starch to Glucose** |
|---|---|---|
| Control, no *Penicillium oxalicum* glucoamylase in pre-saccharification | 8.43 | 3.6% |
| 0.24 AGU /g DS *Penicillium oxalicum* glucoamylase in pre-saccharification | 136.91 | 58.5% |

**Fermentations.** Prior to adding approximately 5g of mash to each tube, each empty tube was weighed. After adding the mashes to the appropriate tubes, all tubes were weighed again to measure the initial mash weights. *Talaromyces emersonii* glucoamylase stock solution was then added to the control tube to an initial dose of 0.50 AGU/g DS whereas no additional glucoamylase was added to the sample which received *Penicillium oxalicum* glucoamylase in the pre-saccharification.

Red Star Ethanol Red yeast from LeSaffre Corp. was rehydrated in tap water at 32°C for 30 minutes (5.50g into 100mLs tap water), and then 100 microL of this suspension was added to each tube using a repeater pipette. Deionized water was also added to each tube to maintain a constant initial %DS. After the *Talaromyces emersonii* glucoamylase, water, and yeast were added to each tube, they were reweighed and then placed into a constant temperature room set at 32°C for a total of 54 hours. Twice per day the tubes were removed from the room, vortexed to mix, reweighed, and then placed back into the room to continue fermentations.

After 54 hours of fermentation, all tubes were vortexed, reweighed, and then the fermentations were stopped by the addition of 50 microL of 40% H₂SO₄ to each tube, followed by thorough vortexing to mix. The tubes were centrifuged at 1500xg for 10 minutes and then the supernatants were syringe-filtered through 0.45 µm nylon filters into labeled HPLC vials. The samples were then analyzed by HPLC for their contents of ethanol.

The results are shown in table 11. It can be seen that *Penicillium oxalicum* glucoamylase when used in the pre-saccharification step at pH 5.80 at 70 °C produced an amount of ethanol equivalent to the amount of ethanol produced by the conventional process (control). This a very good result considering that only half the amount of *Penicillium oxalicum* glucoamylase activity was used compared to the Glucoamylase T glucoamylase activity (0.5 AGU/g DS).

**Table 11**

| **Treatment** | **Ethanol, g/L** |
|---|---|
| Control, no *Penicillium oxalicum* glucoamylase in pre-saccharification, but 0.5 AGU/g DS Glucoamylase T glucoamylase in the fermentation | 110.14 |
| 0.24 AGU /g DS *Penicillium oxalicum* glucoamylase in pre-saccharification | 110.86 |

## Claims

1. A process for producing fermentation products from starch-containing material comprising the steps of:
i) liquefying the starch-containing material using an alpha-amylase;
ii) saccharifying using a glucoamylase having at least 80% identity to the mature polypeptide shown in SEQ ID NO: 9;
iii) fermenting using a fermenting organism.

2. The process of claim 1, wherein the glucoamylase added to saccharification step ii) is a fungal glucoamylase.

3. The process of claim 1 or 2, wherein the glucoamylase added to saccharification step ii) has a heat stability at 70°C, pH 5.3, of at least 80%, preferably at least 85% relative activity.

4. The process of any of claims 1-3, wherein the glucoamylase added to saccharification step ii) has a relative activity at pH 4.5 of at least 80%, preferably at least 85%, preferably at least 90%.

5. The process of any of claims 1-4, wherein the glucoamylase added to saccharification step ii) has a pH stability at pH 4.5 of at least 80%, at least 85%, at least 90%, at least 95%, at least 100% relative activity.

6. The process of any of claims 1-5, wherein the glucoamylase added to saccharification step ii) is disclosed in SEQ ID NO: 9 herein.

7. The process of any of claims 1-6, wherein the glucoamylase added to saccharification step ii) has at least 85%, more preferably at least 90%, more preferably at least 91%, more preferably at least 92%, even more preferably at least 93%, most preferably at least 94%, and even most preferably at least 95%, such as even at least 96%, at least 97%, at least 98%, at least 99% or 100% identity to the mature polypeptide shown in SEQ ID NO: 9 herein.

8. The process of any of claims 1-7, wherein saccharification step ii) is carried out at a temperature from 50°C to 80°C, preferably around 70°C.

9. The process of any of claims 1-8, wherein saccharification step ii) is carried out for 10 minutes to 6 hours, preferably 30 minutes to 3 hours.

10. The process of any of claims 1-9, wherein liquefaction step i) is carried out using a bacterial alpha-amylase or a fungal alpha-amylase.

11. The process of claims 1-10, further comprising, prior to the liquefaction step i), the steps of:
i) milling of starch-containing material;
ii) forming a slurry comprising the milled starch-containing material and water.

12. The process of claim 11, wherein the slurry is heated to above the initial gelatinization temperature.

13. The process of claim 11 or 12, wherein the slurry is jet-cooked at a temperature between 95-140°C, preferably 105-125°C, for 1-15 minutes, preferably for 3-10 minute, especially around 5 minutes, before step i).

14. The process of any of claims 1-13, wherein the alpha-amylase used in liquefaction step i) has a T½ (min) at pH 4.5, 85°C, 0.12 mM CaCl2) of at least 10.

15. The process of claims 1-14, where a protease, preferably a fungal protease, is present during liquefaction step i) or saccharification step ii) and/or fermentation, such as SSF.

16. The process of any of claims 1-15, wherein a protease is a thermostable protease, which has a thermostability value of more than 20% determined as Relative Activity at 80°C/70°C.

17. The process of any of claims 1-16, wherein the protease has a thermostability of more than 30%, more than 40%, more than 50%, more than 60%, more than 70%, more than 80%, more than 90% determined as Relative Activity at 80°C/70°C.

18. The process of claims 1-17, wherein the protease has a thermostability of more than 12%, more than 14%, more than 16%, more than 18%, more than 20%, determined as Relative Activity at 85°C/70°C.

19. The process of any of claims 1-18, further wherein a pullulanase is present during liquefaction step i) and/or saccharification step ii).

## Patentansprüche

1. Verfahren zum Herstellen von Fermentationsprodukten aus stärkehaltigem Material, umfassend die Schritte:
i)Verflüssigen des stärkehaltigen Materials unter Verwendung einer alpha-Amylase;
ii) Verzuckern unter Verwendung einer Glucoamylase mit mindestens 80% Identität zum reifen Polypeptid, das in SEQ ID NO: 9 gezeigt ist;
iii) Fermentieren unter Verwendung eines Fermentationsorganismus.

2. Verfahren nach Anspruch 1, wobei die zum Verzuckerungsschritt ii) zugefügte Glucoamylase eine pilzliche Glucoamylase ist.

3. Verfahren nach Anspruch 1 oder 2, wobei die zum Verzuckerungsschritt ii) zugefügte Glucoamylase eine Hitzestabilität bei 70°C, pH 5,3, von mindestens 80%, bevorzugt mindestens 85% relativer Aktivität aufweist.

4. Verfahren nach einem beliebigen der Ansprüche 1-3, wobei die zum Verzuckerungsschritt ii) zugefügte Glucoamylase eine relative Aktivität bei pH 4,5 von mindestens 80%, bevorzugt mindestens 85%, bevorzugt mindestens 90% relativer Aktivität aufweist.

5. Verfahren nach einem beliebigen der Ansprüche 1-4, wobei die zum Verzuckerungsschritt ii) zugefügte Glucoamylase eine pH-Stabilität bei pH 4,5 von mindestens 80%, mindestens 85%, mindestens 90%, mindestens 95%, mindestens 100% relativer Aktivität aufweist.

6. Verfahren nach einem beliebigen der Ansprüche 1-5, wobei die zum Verzuckerungsschritt ii) zugefügte Glucoamylase hierin in SEQ ID NO: 9 offenbart ist.

7. Verfahren nach einem beliebigen der Ansprüche 1-6, wobei die zum Verzuckerungsschritt ii) zugefügte Glucoamylase mindestens 85%, stärker bevorzugt mindestens 90%, stärker bevorzugt mindestens 91%, stärker bevorzugt mindestens 92%, sogar stärker bevorzugt mindestens 93%, am stärksten bevorzugt mindestens 94% und sogar am stärksten bevorzugt mindestens 95%, wie sogar mindestens 96%, mindestens 97%, mindestens 98%, mindestens 99% oder 100% Identität zum reifen Polypeptid aufweist, das hierein in SEQ ID NO: 9 gezeigt ist.

8. Verfahren nach einem beliebigen der Ansprüche 1-7, wobei Verzuckerungsschritt ii) bei einer Temperatur von 50°C bis 80°C, bevorzugt um 70°C ausgeführt wird.

9. Verfahren nach einem beliebigen der Ansprüche 1-8, wobei Verzuckerungsschritt ii) für 10 Minuten bis 6 Stunden, bevorzugt 30 Minuten bis 3 Stunden ausgeführt wird.

10. Verfahren nach einem beliebigen der Ansprüche 1-9, wobei Verflüssigungsschritt i) unter Verwendung einer bakteriellen alpha-Amylase oder einer pilzlichen alpha-Amylase ausgeführt wird.

11. Verfahren nach Ansprüchen 1-10, weiterhin umfassend vor dem Verflüssigungsschritt i) die Schritte:
i) Mahlen von stärkehaltigem Material;
ii) Bilden einer Aufschlämmung, die das gemahlene stärkehaltige Material und Wasser umfasst.

12. Verfahren nach Anspruch 11, wobei die Aufschlämmung über die anfängliche Gelatinisierungstemperatur erhitzt wird.

13. Verfahren nach Anspruch 11 oder 12, wobei die Aufschlämmung bei einer Temperatur zwischen 95-140°C , bevorzugt 105-125°C, für 1-15 Minuten, bevorzugt 3-10 Minuten, besonders um 5 Minuten vor Schritt i) Strahl-gekocht wird.

14. Verfahren nach einem beliebigen der Ansprüche 1-13, wobei die im Verflüssigungsschritt i) verwendete alpha-Amylase eine T½ (min) bei pH 4,5, 85°C, 0,12 mM CaCl2 von mindestens 10 aufweist.

15. Verfahren nach Ansprüchen 1-14, wobei eine Protease, bevorzugt eine pilzliche Protease, während Verflüssigungsschritt i) oder Verzuckerungsschritt ii) und/oder einer Fermentation vorhanden ist, wie SSF.

16. Verfahren nach einem beliebigen der Ansprüche 1-15, wobei eine Protease eine thermostabile Protease ist, die einen Thermostabilitätswert von mehr als 20% aufweist, bestimmt als relative Aktivität bei 80°C/70°C.

17. Verfahren nach einem beliebigen der Ansprüche 1-16, wobei die Protease eine Thermostabilität von mehr als 30%, mehr als 40%, mehr als 50%, mehr als 60%, mehr als 70%, mehr als 80%, mehr als 90% aufweist, bestimmt als relative Aktivität bei 80°C/70°C.

18. Verfahren nach einem beliebigen der Ansprüche 1-17, wobei die Protease eine Thermostabilität von mehr als 12%, mehr als 14%, mehr als 16%, mehr als 18%, mehr als 20% aufweist, bestimmt als relative Aktivität bei 85°C/70°C.

19. Verfahren nach einem beliebigen der Ansprüche 1-18, wobei weiterhin eine Pullulanase während Verflüssigungsschritt i) und/oder dem Verzuckerungsschritt ii) vorhanden ist.

## Revendications

1. Procédé de production de produits de fermentation à partir de matériau contenant de l'amidon comprenant les étapes suivantes :
i) la liquéfaction du matériau contenant de l'amidon en utilisant une alpha-amylase ;
ii) la saccharification en utilisant une glucoamylase présentant au moins 80 % d'identité avec le polypeptide mature représenté par SEQ ID NO : 9 ;
iii) la fermentation en utilisant un organisme de fermentation.

2. Procédé selon la revendication 1, dans lequel la glucoamylase ajoutée à l'étape de saccharification ii) est une glucoamylase fongique.

3. Procédé selon la revendication 1 ou 2, dans lequel la glucoamylase ajoutée à l'étape de saccharification ii) présente une thermostabilité à 70 °C, pH 5,3, d'au moins 80 %, de préférence d'au moins 85 % d'activité relative.

4. Procédé selon l'une quelconque des revendications 1 à 3, dans lequel la glucoamylase ajoutée à l'étape de saccharification ii) présente une activité relative à pH 4,5 d'au moins 80 %, de préférence d'au moins 85 %, de préférence d'au moins 90 %.

5. Procédé selon l'une quelconque des revendications 1 à 4, dans lequel la glucoamylase ajoutée à l'étape de saccharification ii) présente stabilité au pH à pH 4,5 d'au moins 80 %, d'au moins 85 %, d'au moins 90 %, d'au moins 95 %, d'au moins 100 % d'activité relative.

6. Procédé selon l'une quelconque des revendications 1 à 5, dans lequel la glucoamylase ajoutée à l'étape de saccharification ii) est divulguée par SEQ ID NO : 9 ici.

7. Procédé selon l'une quelconque des revendications 1 à 6, dans lequel la glucoamylase ajoutée à l'étape de saccharification ii) présente au moins 85 %, de façon davantage préférée au moins 90 %, de façon davantage préférée au moins 91 %, de façon davantage préférée au moins 92 %, de façon même davantage préférée au moins 93 %, de façon préférée entre toutes au moins 94 %, et de façon même préférée entre toutes au moins 95 %, comme même au moins 96 %, au moins 97 %, au moins 98 %, au moins 99 % ou 100 % d'identité avec le polypeptide mature représenté par SEQ ID NO : 9 ici.

8. Procédé selon l'une quelconque des revendications 1 à 7, dans lequel l'étape de saccharification ii) est réalisée à une température de 50 °C à 80 °C, de préférence autour de 70 °C.

9. Procédé selon l'une quelconque des revendications 1 à 8, dans lequel l'étape de saccharification ii) est réalisée pendant 10 minutes à 6 heures, de préférence 30 minutes à 3 heures.

10. Procédé selon l'une quelconque des revendications 1 à 9, dans lequel l'étape de liquéfaction i) est réalisée en utilisant une alpha-amylase bactérienne ou une alpha-amylase fongique.

11. Procédé selon les revendications 1 à 10, comprenant en outre avant l'étape de liquéfaction i), les étapes suivantes ;
i) le broyage du matériau contenant de l'amidon ;
ii) la formation d'une suspension épaisse comprenant le matériau contenant de l'amidon broyé et de l'eau.

12. Procédé selon la revendication 11, dans lequel la suspension épaisse est chauffée au-dessus de la température de gélatinisation initiale.

13. Procédé selon la revendication 11 ou 12, dans lequel la suspension épaisse est cuite par jet à une température entre 95 et 140 °C, de préférence de 105 à 125 °C pendant 1 à 15 minutes, de préférence pendant 3 à 10 minutes, spécialement autour de 5 minutes, avant l'étape i).

14. Procédé selon l'une quelconque des revendications 1 à 13, dans lequel l'alpha-amylase utilisée dans l'étape de liquéfaction i) a une T1/2 (min) à pH 4,5, 85 °C, 0,12 mM de CaCl2, d'au moins 10.

15. Procédé selon les revendications 1 à 14, où une protéase, de préférence une protéase fongique, est présente durant l'étape de liquéfaction i) ou l'étape de saccharification ii) et/ou la fermentation, telle que la SSF.

16. Procédé selon l'une quelconque des revendications 1 à 15, dans lequel une protéase est une protéase thermostable, qui a une valeur de thermostabilité de plus de 20 % déterminée comme l'activité relative à 80 °C/70 °C.

17. Procédé selon l'une quelconque des revendications 1 à 16, dans lequel la protéase a une thermostabilité de plus de 30 %, de plus de 40 %, de plus de 50 %, de plus de 60 %, de plus de 70 %, de plus de 80 %, de plus de 90 % déterminée comme l'activité relative à 80 °C/70 °C.

18. Procédé selon les revendications 1 à 17, dans lequel la protéase a une thermostabilité de plus de 12 %, de plus de 14 %, de plus de 16 %, de plus de 18 %, de plus de 20 %, déterminée comme l'activité relative à 85 °C/70 °C.

19. Procédé selon l'une quelconque des revendications 1 à 18, dans lequel en outre une pullulanase est présente durant l'étape de liquéfaction i) et/ou l'étape de saccharification ii).


### SEQUENCE MISTING

<110> Novozymes A/S Deinhammer, Randy Li, Ming Duan, Junxin Lieu, Zheng Fukuyama, Shiro Ayabe, Keiichi Coward-Kelly, Guillermo
<120> Processes for Producing Fermentation Products
<130> 12134-CN-PCT
<160> 12
<170> PatentIn version 3.5
<210> 1
   <211> 515
   <212> PRT
   <213> Bacillus stearothermophilus
<220>
   <221> mat_peptide
   <222> (1)..(515)
<400> 1
   Ala Ala Pro Phe Asn Gly Thr Met Met Gln Tyr Phe Glu Trp Tyr Leu
   1               5                   10                  15
   Pro Asp Asp Gly Thr Leu Trp Thr Lys Val Ala Asn Glu Ala Asn Asn
               20                  25                  30
   Leu Ser Ser Leu Gly Ile Thr Ala Leu Trp Leu Pro Pro Ala Tyr Lys
           35                  40                  45
   Gly Thr Ser Arg Ser Asp Val Gly Tyr Gly Val Tyr Asp Leu Tyr Asp
       50                  55                  60
   Leu Gly Glu Phe Asn Gln Lys Gly Thr Val Arg Thr Lys Tyr Gly Thr
   65                  70                  75                  80
   Lys Ala Gln Tyr Leu Gln Ala Ile Gln Ala Ala His Ala Ala Gly Met
                   85                  90                  95
   Gln Val Tyr Ala Asp Val Val Phe Asp His Lys Gly Gly Ala Asp Gly
               100                 105                 110
   Thr Glu Trp Val Asp Ala Val Glu Val Asn Pro Ser Asp Arg Asn Gln
           115                 120                 125
   Glu Ile Ser Gly Thr Tyr Gln Ile Gln Ala Trp Thr Lys Phe Asp Phe
       130                 135                 140
   Pro Gly Arg Gly Asn Thr Tyr Ser Ser Phe Lys Trp Arg Trp Tyr His
   145                 150                 155                 160
   Phe Asp Gly Val Asp Trp Asp Glu Ser Arg Lys Leu Ser Arg Ile Tyr
                   165                 170                 175
   Lys Phe Arg Gly Ile Gly Lys Ala Trp Asp Trp Glu Val Asp Thr Glu
               180                 185                 190
   Asn Gly Asn Tyr Asp Tyr Leu Met Tyr Ala Asp Leu Asp Met Asp His
           195                 200                 205
   Pro Glu Val Val Thr Glu Leu Lys Asn Trp Gly Lys Trp Tyr Val Asn
       210                 215                 220
   Thr Thr Asn Ile Asp Gly Phe Arg Leu Asp Ala Val Lys His Ile Lys
   225                 230                 235                 240
   Phe Ser Phe Phe Pro Asp Trp Leu Ser Tyr Val Arg Ser Gln Thr Gly
                   245                 250                 255
   Lys Pro Leu Phe Thr Val Gly Glu Tyr Trp Ser Tyr Asp Ile Asn Lys
               260                 265                 270
   Leu His Asn Tyr Ile Thr Lys Thr Asn Gly Thr Met Ser Leu Phe Asp
           275                 280                 285
   Ala Pro Leu His Asn Lys Phe Tyr Thr Ala Ser Lys Ser Gly Gly Ala
       290                 295                 300
   Phe Asp Met Arg Thr Leu Met Thr Asn Thr Leu Met Lys Asp Gln Pro
   305                 310                 315                 320
   Thr Leu Ala Val Thr Phe Val Asp Asn His Asp Thr Glu Pro Gly Gln
                   325                 330                 335
   Ala Leu Gln Ser Trp Val Asp Pro Trp Phe Lys Pro Leu Ala Tyr Ala
               340                 345                 350
   Phe Ile Leu Thr Arg Gln Glu Gly Tyr Pro Cys Val Phe Tyr Gly Asp
           355                 360                 365
   Tyr Tyr Gly Ile Pro Gln Tyr Asn Ile Pro Ser Leu Lys Ser Lys Ile
       370                 375                 380
   Asp Pro Leu Leu Ile Ala Arg Arg Asp Tyr Ala Tyr Gly Thr Gln His
   385                 390                 395                 400
   Asp Tyr Leu Asp His Ser Asp Ile Ile Gly Trp Thr Arg Glu Gly Val
                   405                 410                 415
   Thr Glu Lys Pro Gly Ser Gly Leu Ala Ala Leu Ile Thr Asp Gly Pro
               420                 425                 430
   Gly Gly Ser Lys Trp Met Tyr Val Gly Lys Gln His Ala Gly Lys Val
           435                 440                 445
   Phe Tyr Asp Leu Thr Gly Asn Arg Ser Asp Thr Val Thr Ile Asn Ser
       450                 455                 460
   Asp Gly Trp Gly Glu Phe Lys Val Asn Gly Gly Ser Val Ser Val Trp
   465                 470                 475                 480
   Val Pro Arg Lys Thr Thr Val Ser Thr Ile Ala Arg Pro Ile Thr Thr
                   485                 490                 495
   Arg Pro Trp Thr Gly Glu Phe Val Arg Trp Thr Glu Pro Arg Leu Val
               500                 505                 510
   Ala Trp Pro
           515
<210> 2
   <211> 1068
   <212> DNA
   <213> Thermoascus aurantiacus
<220>
   <221> CDS
   <222> (1)..(1065)
<220>
   <221> misc_signal
   <222> (1)..(57)
<220>
   <221> misc_feature
   <222> (58)..(534)
<220>
   <221> mat_peptide
   <222> (535)..(1068)
<400> 2
   atg cgg ctc gtt  gct tcc cta acg gcc  ttg gtg gcc ttg tcc  gta        45
   Met Arg Leu Val  Ala Ser Leu Thr Ala  Leu Val Ala Leu Ser  Val
               -175                 -170                 -165
   cct gtc ttt ccc  gct gct gtc aac gtg  aag cgt gct tcg tcc  tac        90
   Pro Val Phe Pro  Ala Ala Val Asn Val  Lys Arg Ala Ser Ser  Tyr
               -160                 -155                 -150
   ctg gag atc act  ctg agc cag gtc agc  aac act ctg atc aag  gcc       135
   Leu Glu Ile Thr  Leu Ser Gln Val Ser  Asn Thr Leu Ile Lys  Ala
               -145                 -140                 -135
   gtg gtc cag aac  act ggt agc gac gag  ttg tcc ttc gtt cac  ctg        180
   Val Val Gln Asn  Thr Gly Ser Asp Glu  Leu Ser Phe Val His  Leu
               -130                 -125                 -120
   aac ttc ttc aag  gac ccc gct cct gtc  aaa aag gta tcg gtc  tat        225
   Asn Phe Phe Lys  Asp Pro Ala Pro Val  Lys Lys Val Ser Val  Tyr
               -115                 -110                 -105
   cgc gat ggg tct  gaa gtg cag ttc gag ggc att ttg agc cgc tac aaa      273
   Arg Asp Gly Ser  Glu Val Gln Phe Glu Gly Ile Leu Ser Arg Tyr Lys
               -100                 -95                 -90
   tcg act ggc ctc tct cgt gac gcc ttt act tat ctg gct ccc gga gag       321
   Ser Thr Gly Leu Ser Arg Asp Ala Phe Thr Tyr Leu Ala Pro Gly Glu
           -85                 -80                 -75
   tcc gtc gag gac gtt ttt gat att gct tcg act tac gat ctg acc agc       369
   Ser Val Glu Asp Val Phe Asp Ile Ala Ser Thr Tyr Asp Leu Thr Ser
       -70                 -65                 -60
   ggc ggc cct gta act atc cgt act gag gga gtt gtt ccc tac gcc acg       417
   Gly Gly Pro Val Thr Ile Arg Thr Glu Gly Val Val Pro Tyr Ala Thr
   -55                 -50                 -45                 -40
   gct aac agc act gat att gcc ggc tac atc tca tac tcg tct aat gtg       465
   Ala Asn Ser Thr Asp Ile Ala Gly Tyr Ile Ser Tyr Ser Ser Asn Val
                   -35                 -30                 -25
   ttg acc att gat gtc gat ggc gcc gct gct gcc act gtc tcc aag gca       513
   Leu Thr Ile Asp Val Asp Gly Ala Ala Ala Ala Thr Val Ser Lys Ala
               -20                 -15                 -10
   atc act cct ttg gac cgc cgc act agg atc agt tcc tgc tcc ggc agc       561
   Ile Thr Pro Leu Asp Arg Arg Thr Arg Ile Ser Ser Cys Ser Gly Ser
           -5                  -1  1               5
   aga cag agc gct ctt act acg gct ctc aga aac gct gct tct ctt gcc       609
   Arg Gln Ser Ala Leu Thr Thr Ala Leu Arg Asn Ala Ala Ser Leu Ala
   10                  15                  20                  25
   aac gca gct gcc gac gcg gct cag tct gga tca gct tca aag ttc agc       657
   Asn Ala Ala Ala Asp Ala Ala Gln Ser Gly Ser Ala Ser Lys Phe Ser
                   30                  35                  40
   gag tac ttc aag act act tct agc tct acc cgc cag acc gtg gct gcg       705
   Glu Tyr Phe Lys Thr Thr Ser Ser Ser Thr Arg Gln Thr Val Ala Ala
               45                  50                  55
   cgt ctt cgg gct gtt gcg cgg gag gca tct tcg tct tct tcg gga gcc       753
   Arg Leu Arg Ala Val Ala Arg Glu Ala Ser Ser Ser Ser Ser Gly Ala
           60                  65                  70
   acc acg tac tac tgc gac gat ccc tac ggc tac tgt tcc tcc aac gtc       801
   Thr Thr Tyr Tyr Cys Asp Asp Pro Tyr Gly Tyr Cys Ser Ser Asn Val
       75                  80                  85
   ctg gct tac acc ctg cct tca tac aac ata atc gcc aac tgt gac att       849
   Leu Ala Tyr Thr Leu Pro Ser Tyr Asn Ile Ile Ala Asn Cys Asp Ile
   90                  95                  100                 105
   ttc tat act tac ctg ccg gct ctg acc agt acc tgt cac gct cag gat       897
   Phe Tyr Thr Tyr Leu Pro Ala Leu Thr Ser Thr Cys His Ala Gln Asp
                   110                 115                 120
   caa gcg acc act gcc ctt cac gag ttc acc cat gcg cct ggc gtc tac           945
   Gln Ala Thr Thr Ala Leu His Glu Phe Thr His Ala Pro Gly Val Tyr
               125                 130                 135
   agc cct ggc acg gac gac ctg gcg tat ggc tac cag gct gcg atg ggt           993
   Ser Pro Gly Thr Asp Asp Leu Ala Tyr Gly Tyr Gln Ala Ala Met Gly
           140                 145                 150
   ctc agc agc agc cag gct gtc atg aac gct gac acc tac gct ctc tat          1041
   Leu Ser Ser Ser Gln Ala Val Met Asn Ala Asp Thr Tyr Ala Leu Tyr
       155                 160                 165
   gcg aat gcc ata tac ctt ggt tgc taa                                      1068
   Ala Asn Ala Ile Tyr Leu Gly Cys
   170                 175
<210> 3
   <211> 355
   <212> PRT
   <213> Thermoascus aurantiacus
<400> 3
   Met Arg Leu Val  Ala Ser Leu Thr Ala  Leu Val Ala Leu Ser  Val
               -175                 -170                 -165
   Pro Val Phe Pro  Ala Ala Val Asn Val  Lys Arg Ala Ser Ser  Tyr
               -160                 -155                 -150
   Leu Glu Ile Thr  Leu Ser Gln Val Ser  Asn Thr Leu Ile Lys  Ala
               -145                 -140                 -135
   Val Val Gln Asn  Thr Gly Ser Asp Glu  Leu Ser Phe Val His  Leu
               -130                 -125                 -120
   Asn Phe Phe Lys  Asp Pro Ala Pro Val  Lys Lys Val Ser Val  Tyr
               -115                 -110                 -105
   Arg Asp Gly Ser  Glu Val Gln Phe Glu Gly Ile Leu Ser Arg Tyr Lys
               -100                 -95                 -90
   Ser Thr Gly Leu Ser Arg Asp Ala Phe Thr Tyr Leu Ala Pro Gly Glu
           -85                 -80                 -75
   Ser Val Glu Asp Val Phe Asp Ile Ala Ser Thr Tyr Asp Leu Thr Ser
       -70                 -65                 -60
   Gly Gly Pro Val Thr Ile Arg Thr Glu Gly Val Val Pro Tyr Ala Thr
   -55                 -50                 -45                 -40
   Ala Asn Ser Thr Asp Ile Ala Gly Tyr Ile Ser Tyr Ser Ser Asn Val
                   -35                 -30                 -25
   Leu Thr Ile Asp Val Asp Gly Ala Ala Ala Ala Thr Val Ser Lys Ala
               -20                 -15                 -10
   Ile Thr Pro Leu Asp Arg Arg Thr Arg Ile Ser Ser Cys Ser Gly Ser
           -5                  -1  1                   5
   Arg Gln Ser Ala Leu Thr Thr Ala Leu Arg Asn Ala Ala Ser Leu Ala
   10                  15                  20                  25
   Asn Ala Ala Ala Asp Ala Ala Gln Ser Gly Ser Ala Ser Lys Phe Ser
                   30                  35                  40
   Glu Tyr Phe Lys Thr Thr Ser Ser Ser Thr Arg Gln Thr Val Ala Ala
               45                  50                  55
   Arg Leu Arg Ala Val Ala Arg Glu Ala Ser Ser Ser Ser Ser Gly Ala
           60                  65                  70
   Thr Thr Tyr Tyr Cys Asp Asp Pro Tyr Gly Tyr Cys Ser Ser Asn Val
       75                  80                  85
   Leu Ala Tyr Thr Leu Pro Ser Tyr Asn Ile Ile Ala Asn Cys Asp Ile
   90                  95                  100                 105
   Phe Tyr Thr Tyr Leu Pro Ala Leu Thr Ser Thr Cys His Ala Gln Asp
                   110                 115                 120
   Gln Ala Thr Thr Ala Leu His Glu Phe Thr His Ala Pro Gly Val Tyr
               125                 130                 135
   Ser Pro Gly Thr Asp Asp Leu Ala Tyr Gly Tyr Gln Ala Ala Met Gly
           140                 145                 150
   Leu Ser Ser Ser Gln Ala Val Met Asn Ala Asp Thr Tyr Ala Leu Tyr
       155                 160                 165
   Ala Asn Ala Ile Tyr Leu Gly Cys
   170                 175
<210> 4
   <211> 49
   <212> DNA
   <213> Artificial Sequence
<220>
   <223> Synthetic Construct
<400> 4
   aacgacggta cccggggatc ggatccatgc ggctcgttgc ttccctaac 49
<210> 5
   <211> 48
   <212> DNA
   <213> Artificial Sequence
<220>
   <223> Artificial Construct
<400> 5
   ctaattacat gatgcggccc ttaattaatt agcaaccaag gtatatgg 48
<210> 6
   <211> 20
   <212> DNA
   <213> Artificial Sequence
<220>
   <223> Artificial Construct
<400> 6
   taggagttta gtgaacttgc 20
<210> 7
   <211> 18
   <212> DNA
   <213> Artificial Sequence
<220>
   <223> Artificial Construct
<400> 7
   ttcgagcgtc ccaaaacc 18
<210> 8
   <211> 1851
   <212> DNA
   <213> Penicillium oxalicum
<220>
   <221> CDS
   <222> (1)..(1851)
<400> 8
   atg cgt ctc act cta tta tca ggt gta gcc ggc gtt ctc tgc gca gga       48
   Met Arg Leu Thr Leu Leu Ser Gly Val Ala Gly Val Leu Cys Ala Gly
   1               5                   10                  15
   cag ctg acg gcg gcg cgt cct gat ccc aag ggt ggg aat ctg acg ccg       96
   Gln Leu Thr Ala Ala Arg Pro Asp Pro Lys Gly Gly Asn Leu Thr Pro
               20                  25                  30
   ttc atc cac aaa gag ggc gag cgg tcg ctc caa ggc atc ttg gac aat      144
   Phe Ile His Lys Glu Gly Glu Arg Ser Leu Gln Gly Ile Leu Asp Asn
           35                  40                  45
   ctc ggt ggg cga ggt aag aaa aca ccc ggc act gcc gca ggg ttg ttt      192
   Leu Gly Gly Arg Gly Lys Lys Thr Pro Gly Thr Ala Ala Gly Leu Phe
       50                  55                  60
   att gcc agt cca aac aca gag aat cca aac tat tat tat aca tgg act      240
   Ile Ala Ser Pro Asn Thr Glu Asn Pro Asn Tyr Tyr Tyr Thr Trp Thr
   65                  70                  75                  80
   cgt gac tca gct ttg act gcc aag tgc ttg atc gac ctg ttc gaa gac          288
   Arg Asp Ser Ala Leu Thr Ala Lys Cys Leu Ile Asp Leu Phe Glu Asp
                   85                  90                  95
   tct cgg gca aag ttt cca att gac cgc aaa tac ttg gaa aca gga att          336
   Ser Arg Ala Lys Phe Pro Ile Asp Arg Lys Tyr Leu Glu Thr Gly Ile
               100                 105                 110
   cgg gac tac gtg tcg tcc caa gca atc ctc cag agt gtg tct aat cct          384
   Arg Asp Tyr Val Ser Ser Gln Ala Ile Leu Gln Ser Val Ser Asn Pro
           115                 120                 125
   tct gga acc ctg aag gat ggc tct ggt ctg ggt gaa ccc aag ttt gag          432
   Ser Gly Thr Leu Lys Asp Gly Ser Gly Leu Gly Glu Pro Lys Phe Glu
       130                 135                 140
   att gac ctg aat ccc ttt tcg ggt gcc tgg ggt cgg cct cag cgg gat          480
   Ile Asp Leu Asn Pro Phe Ser Gly Ala Trp Gly Arg Pro Gln Arg Asp
   145                 150                 155                 160
   ggc cca gcg ctg cga gcg acc gct atg atc acc tac gcc aac tac ctg          528
   Gly Pro Ala Leu Arg Ala Thr Ala Met Ile Thr Tyr Ala Asn Tyr Leu
                   165                 170                 175
   ata tcc cat ggt cag aaa tcg gat gtg tca cag gtc atg tgg ccg att          576
   Ile Ser His Gly Gln Lys Ser Asp Val Ser Gln Val Met Trp Pro Ile
               180                 185                 190
   att gcc aat gat cta gca tat gtt ggt caa tac tgg aat aat acc gga          624
   Ile Ala Asn Asp Leu Ala Tyr Val Gly Gln Tyr Trp Asn Asn Thr Gly
           195                 200                 205
   ttt gac ctg tgg gaa gag gtg gat ggg tca agc ttt ttc acg att gcg          672
   Phe Asp Leu Trp Glu Glu Val Asp Gly Ser Ser Phe Phe Thr Ile Ala
       210                 215                 220
   gtc cag cac cga gcc ctt gtt gaa ggc tcg caa ctg gcg aaa aag ctc          720
   Val Gln His Arg Ala Leu Val Glu Gly Ser Gln Leu Ala Lys Lys Leu
   225                 230                 235                 240
   ggc aag tcc tgc gat gcc tgt gat tct cag cct ccc cag ata ttg tgt          768
   Gly Lys Ser Cys Asp Ala Cys Asp Ser Gln Pro Pro Gln Ile Leu Cys
                   245                 250                 255
   ttc ctg cag agt ttc tgg aac gga aag tac atc acc tcc aac atc aac          816
   Phe Leu Gln Ser Phe Trp Asn Gly Lys Tyr Ile Thr Ser Asn Ile Asn
               260                 265                 270
   acg caa gca agc cgc tct ggt atc gac ctg gac tct gtc ctg gga agc          864
   Thr Gln Ala Ser Arg Ser Gly Ile Asp Leu Asp Ser Val Leu Gly Ser
           275                 280                 285
   att cat acc ttt gat ccc gaa gca gcc tgt gac gat gca act ttc cag          912
   Ile His Thr Phe Asp Pro Glu Ala Ala Cys Asp Asp Ala Thr Phe Gln
       290                 295                 300
   cct tgt tct gcc cgc gct ctg gcg aac cac aag gtc tat gtg gat tcc          960
   Pro Cys Ser Ala Arg Ala Leu Ala Asn His Lys Val Tyr Val Asp Ser
   305                 310                 315                 320
   ttc cgc tct atc tac aag att aat gcg ggt ctt gca gag gga tcg gct         1008
   Phe Arg Ser Ile Tyr Lys Ile Asn Ala Gly Leu Ala Glu Gly Ser Ala
                   325                 330                 335
   gcc aac gtt ggc cgc tac ccc gag gat gtt tac caa gga ggc aat cca        1056
   Ala Asn Val Gly Arg Tyr Pro Glu Asp Val Tyr Gln Gly Gly Asn Pro
               340                 345                 350
   tgg tat ctc gcc acc cta ggc gca tct gaa ttg ctt tac gac gcc ttg        1104
   Trp Tyr Leu Ala Thr Leu Gly Ala Ser Glu Leu Leu Tyr Asp Ala Leu
           355                 360                 365
   tac cag tgg gac aga ctt ggc aaa ctt gaa gtc tcg gag acc tcg ttg        1152
   Tyr Gln Trp Asp Arg Leu Gly Lys Leu Glu Val Ser Glu Thr Ser Leu
       370                 375                 380
   tca ttc ttc aaa gac ttt gac gcg acc gtg aaa att ggc tcg tac tcg        1200
   Ser Phe Phe Lys Asp Phe Asp Ala Thr Val Lys Ile Gly Ser Tyr Ser
   385                 390                 395                 400
   agg aac agc aag acc tac aag aaa ttg acc cag tcc atc aag tcg tac        1248
   Arg Asn Ser Lys Thr Tyr Lys Lys Leu Thr Gln Ser Ile Lys Ser Tyr
                   405                 410                 415
   gcg gac ggg ttc atc cag tta gtg cag cag tac act cct tct aat gga        1296
   Ala Asp Gly Phe Ile Gln Leu Val Gln Gln Tyr Thr Pro Ser Asn Gly
               420                 425                 430
   tct ctg gcc gag caa tac gat cgc aat acg gct gct cct ctc tct gca        1344
   Ser Leu Ala Glu Gln Tyr Asp Arg Asn Thr Ala Ala Pro Leu Ser Ala
           435                 440                 445
   aac gat ctg act tgg tca ttt gcc tct ttc ttg acg gct acg caa cgc        1392
   Asn Asp Leu Thr Trp Ser Phe Ala Ser Phe Leu Thr Ala Thr Gln Arg
       450                 455                 460
   cgc gat gcc gtg gtt cct ccc tcc tgg ggc gca aag tcg gca aac aaa        1440
   Arg Asp Ala Val Val Pro Pro Ser Trp Gly Ala Lys Ser Ala Asn Lys
   465                 470                 475                 480
   gtc cca acc act tgt tca gcc tcc cct gtt gtg ggt act tat aag gcg        1488
   Val Pro Thr Thr Cys Ser Ala Ser Pro Val Val Gly Thr Tyr Lys Ala
                   485                 490                 495
   ccc acg gca act ttc tca tcc aag act aag tgc gtc ccc gct aaa gat        1536
   Pro Thr Ala Thr Phe Ser Ser Lys Thr Lys Cys Val Pro Ala Lys Asp
               500                 505                 510
   att gtg cct atc acg ttc tac ctg att gag aac act tac tat gga gag        1584
   Ile Val Pro Ile Thr Phe Tyr Leu Ile Glu Asn Thr Tyr Tyr Gly Glu
           515                 520                 525
   aac gtc ttc atg agt ggc aac att act gcg ctg ggt aac tgg gac gcc        1632
   Asn Val Phe Met Ser Gly Asn Ile Thr Ala Leu Gly Asn Trp Asp Ala
       530                 535                 540
   aag aaa ggc ttc cca ctc acc gca aac ctc tac acg caa gat caa aac        1680
   Lys Lys Gly Phe Pro Leu Thr Ala Asn Leu Tyr Thr Gln Asp Gln Asn
   545                 550                 555                 560
   ttg tgg ttc gcc agt gtc gag ttc atc cca gca ggc aca ccc ttt gag        1728
   Leu Trp Phe Ala Ser Val Glu Phe Ile Pro Ala Gly Thr Pro Phe Glu
                   565                 570                 575
   tac aag tac tac aag gtc gag ccc aat ggc gat att act tgg gag aag        1776
   Tyr Lys Tyr Tyr Lys Val Glu Pro Asn Gly Asp Ile Thr Trp Glu Lys
               580                 585                 590
   ggt ccc aac cgg gtg ttc gtc gct ccc acg gga tgc cca gtt cag cct      1824
   Gly Pro Asn Arg Val Phe Val Ala Pro Thr Gly Cys Pro Val Gln Pro
           595                 600                 605
   cac tcc aac gac gtg tgg cag ttt tga                                  1851
   His Ser Asn Asp Val Trp Gln Phe
       610                 615
<210> 9
   <211> 616
   <212> PRT
   <213> Penicillium oxalicum
<400> 9
   Met Arg Leu Thr Leu Leu Ser Gly Val Ala Gly Val Leu Cys Ala Gly
   1               5                   10                  15
   Gln Leu Thr Ala Ala Arg Pro Asp Pro Lys Gly Gly Asn Leu Thr Pro
               20                  25                  30
   Phe Ile His Lys Glu Gly Glu Arg Ser Leu Gln Gly Ile Leu Asp Asn
           35                  40                  45
   Leu Gly Gly Arg Gly Lys Lys Thr Pro Gly Thr Ala Ala Gly Leu Phe
       50                  55                  60
   Ile Ala Ser Pro Asn Thr Glu Asn Pro Asn Tyr Tyr Tyr Thr Trp Thr
   65                  70                  75                  80
   Arg Asp Ser Ala Leu Thr Ala Lys Cys Leu Ile Asp Leu Phe Glu Asp
                   85                  90                  95
   Ser Arg Ala Lys Phe Pro Ile Asp Arg Lys Tyr Leu Glu Thr Gly Ile
               100                 105                 110
   Arg Asp Tyr Val Ser Ser Gln Ala Ile Leu Gln Ser Val Ser Asn Pro
           115                 120                 125
   Ser Gly Thr Leu Lys Asp Gly Ser Gly Leu Gly Glu Pro Lys Phe Glu
       130                 135                 140
   Ile Asp Leu Asn Pro Phe Ser Gly Ala Trp Gly Arg Pro Gln Arg Asp
   145                 150                 155                 160
   Gly Pro Ala Leu Arg Ala Thr Ala Met Ile Thr Tyr Ala Asn Tyr Leu
                   165                 170                 175
   Ile Ser His Gly Gln Lys Ser Asp Val Ser Gln Val Met Trp Pro Ile
               180                 185                 190
   Ile Ala Asn Asp Leu Ala Tyr Val Gly Gln Tyr Trp Asn Asn Thr Gly
   195 200 205
   Phe Asp Leu Trp Glu Glu Val Asp Gly Ser Ser Phe Phe Thr Ile Ala
   210 215 220
   Val Gln His Arg Ala Leu Val Glu Gly Ser Gln Leu Ala Lys Lys Leu
   225 230 235 240
   Gly Lys Ser Cys Asp Ala Cys Asp Ser Gln Pro Pro Gln Ile Leu Cys
   245 250 255
   Phe Leu Gln Ser Phe Trp Asn Gly Lys Tyr Ile Thr Ser Asn Ile Asn
   260 265 270
   Thr Gln Ala Ser Arg Ser Gly Ile Asp Leu Asp Ser Val Leu Gly Ser
   275 280 285
   Ile His Thr Phe Asp Pro Glu Ala Ala Cys Asp Asp Ala Thr Phe Gln
   290 295 300
   Pro Cys Ser Ala Arg Ala Leu Ala Asn His Lys Val Tyr Val Asp Ser
   305 310 315 320
   Phe Arg Ser Ile Tyr Lys Ile Asn Ala Gly Leu Ala Glu Gly Ser Ala
   325 330 335
   Ala Asn Val Gly Arg Tyr Pro Glu Asp Val Tyr Gln Gly Gly Asn Pro
   340 345 350
   Trp Tyr Leu Ala Thr Leu Gly Ala Ser Glu Leu Leu Tyr Asp Ala Leu
   355 360 365
   Tyr Gln Trp Asp Arg Leu Gly Lys Leu Glu Val Ser Glu Thr Ser Leu
   370 375 380
   Ser Phe Phe Lys Asp Phe Asp Ala Thr Val Lys Ile Gly Ser Tyr Ser
   385 390 395 400
   Arg Asn Ser Lys Thr Tyr Lys Lys Leu Thr Gln Ser Ile Lys Ser Tyr
   405 410 415
   Ala Asp Gly Phe Ile Gln Leu Val Gln Gln Tyr Thr Pro Ser Asn Gly
   420 425 430
   Ser Leu Ala Glu Gln Tyr Asp Arg Asn Thr Ala Ala Pro Leu Ser Ala
   435 440 445
   Asn Asp Leu Thr Trp Ser Phe Ala Ser Phe Leu Thr Ala Thr Gln Arg
   450 455 460
   Arg Asp Ala Val Val Pro Pro Ser Trp Gly Ala Lys Ser Ala Asn Lys
   465                 470                 475                 480
   Val Pro Thr Thr Cys Ser Ala Ser Pro Val Val Gly Thr Tyr Lys Ala
                   485                 490                 495
   Pro Thr Ala Thr Phe Ser Ser Lys Thr Lys Cys Val Pro Ala Lys Asp
               500                 505                 510
   Ile Val Pro Ile Thr Phe Tyr Leu Ile Glu Asn Thr Tyr Tyr Gly Glu
           515                 520                 525
   Asn Val Phe Met Ser Gly Asn Ile Thr Ala Leu Gly Asn Trp Asp Ala
       530                 535                 540
   Lys Lys Gly Phe Pro Leu Thr Ala Asn Leu Tyr Thr Gln Asp Gln Asn
   545                 550                 555                 560
   Leu Trp Phe Ala Ser Val Glu Phe Ile Pro Ala Gly Thr Pro Phe Glu
                   565                 570                 575
   Tyr Lys Tyr Tyr Lys Val Glu Pro Asn Gly Asp Ile Thr Trp Glu Lys
               580                 585                 590
   Gly Pro Asn Arg Val Phe Val Ala Pro Thr Gly Cys Pro Val Gln Pro
           595                 600                 605
   His Ser Asn Asp Val Trp Gln Phe
       610                 615
<210> 11
   <211> 1337
   <212> PRT
   <213> Thermococcus hydrothermalis
<400> 11
   Met Arg Arg Val Val Ala Leu Phe Ile Ala Ile Leu Met Leu Gly Ser
           -25                 -20                 -15
   Ile Val Gly Ala Asn Val Lys Ser Val Gly Ala Ala Glu Pro Lys Pro
       -10                 -5                  -1  1               5
   Leu Asn Val Ile Ile Val Trp His Gln His Gln Pro Tyr Tyr Tyr Asp
                   10                  15                  20
   Pro Val Gln Asp Val Tyr Thr Arg Pro Trp Val Arg Leu His Ala Ala
               25                  30                  35
   Asn Asn Tyr Trp Lys Met Ala His Tyr Leu Ser Gln Tyr Pro Glu Val
           40                  45                  50
   His Ala Thr Ile Asp Leu Ser Gly Ser Leu Ile Ala Gln Leu Ala Asp
       55                  60                  65
   Tyr Met Asn Gly Lys Lys Asp Thr Tyr Gln Ile Ile Thr Glu Lys Ile
   70                  75                  80                  85
   Ala Asn Gly Glu Pro Leu Thr Val Asp Glu Lys Trp Phe Met Leu Gln
                   90                  95                  100
   Ala Pro Gly Gly Phe Phe Asp Asn Thr Ile Pro Trp Asn Gly Glu Pro
               105                 110                 115
   Ile Thr Asp Pro Asn Gly Asn Pro Ile Arg Asp Phe Trp Asp Arg Tyr
           120                 125                 130
   Thr Glu Leu Lys Asn Lys Met Leu Ser Ala Lys Ala Lys Tyr Ala Asn
       135                 140                 145
   Phe Val Thr Glu Ser Gln Lys Val Ala Val Thr Asn Glu Phe Thr Glu
   150                 155                 160                 165
   Gln Asp Tyr Ile Asp Leu Ala Val Leu Phe Asn Leu Ala Trp Ile Asp
                   170                 175                 180
   Tyr Asn Tyr Ile Thr Ser Thr Pro Glu Phe Lys Ala Leu Tyr Asp Lys
               185                 190                 195
   Val Asp Glu Gly Gly Tyr Thr Arg Ala Asp Val Lys Thr Val Leu Asp
           200                 205                 210
   Ala Gln Ile Trp Leu Leu Asn His Thr Phe Glu Glu His Glu Lys Ile
       215                 220                 225
   Asn Leu Leu Leu Gly Asn Gly Asn Val Glu Val Thr Val Val Pro Tyr
   230                 235                 240                 245
   Ala His Pro Ile Gly Pro Ile Leu Asn Asp Phe Gly Trp Asp Ser Asp
                   250                 255                 260
   Phe Asn Asp Gln Val Lys Lys Ala Asp Glu Leu Tyr Lys Pro Tyr Leu
               265                 270                 275
   Gly Gly Gly Thr Ala Val Pro Lys Gly Gly Trp Ala Ala Glu Ser Ala
           280                 285                 290
   Leu Asn Asp Lys Thr Leu Glu Ile Leu Ala Glu Asn Gly Trp Glu Trp
       295                 300                 305
   Val Met Thr Asp Gln Met Val Leu Gly Lys Leu Gly Ile Glu Gly Thr
   310                 315                 320                 325
   Val Glu Asn Tyr His Lys Pro Trp Val Ala Glu Phe Asn Gly Lys Lys
                   330                 335                 340
   Ile Tyr Leu Phe Pro Arg Asn His Asp Leu Ser Asp Arg Val Gly Phe
               345                 350                 355
   Thr Tyr Ser Gly Met Asn Gln Gln Gln Ala Val Glu Asp Phe Val Asn
           360                 365                 370
   Glu Leu Leu Lys Leu Gln Lys Gln Asn Tyr Asp Gly Ser Leu Val Tyr
       375                 380                 385
   Val Val Thr Leu Asp Gly Glu Asn Pro Val Glu Asn Tyr Pro Tyr Asp
   390                 395                 400                 405
   Gly Glu Leu Phe Leu Thr Glu Leu Tyr Lys Lys Leu Thr Glu Leu Gln
                   410                 415                 420
   Glu Gln Gly Leu Ile Arg Thr Leu Thr Pro Ser Glu Tyr Ile Gln Leu
               425                 430                 435
   Tyr Gly Asp Lys Ala Asn Lys Leu Thr Pro Arg Met Met Glu Arg Leu
           440                 445                 450
   Asp Leu Thr Gly Asp Asn Val Asn Ala Leu Leu Lys Ala Gln Ser Leu
       455                 460                 465
   Gly Glu Leu Tyr Asp Met Thr Gly Val Lys Glu Glu Met Gln Trp Pro
   470                 475                 480                 485
   Glu Ser Ser Trp Ile Asp Gly Thr Leu Ser Thr Trp Ile Gly Glu Pro
                   490                 495                 500
   Gln Glu Asn Tyr Gly Trp Tyr Trp Leu Tyr Met Ala Arg Lys Ala Leu
               505                 510                 515
   Met Glu Asn Lys Asp Lys Met Ser Gln Ala Asp Trp Glu Lys Ala Tyr
           520                 525                 530
   Glu Tyr Leu Leu Arg Ala Glu Ala Ser Asp Trp Phe Trp Trp Tyr Gly
       535                 540                 545
   Ser Asp Gln Asp Ser Gly Gln Asp Tyr Thr Phe Asp Arg Tyr Leu Lys
   550                 555                 560                 565
   Thr Tyr Leu Tyr Glu Met Tyr Lys Leu Ala Gly Val Glu Pro Pro Ser
                   570                 575                 580
   Tyr Leu Phe Gly Asn Tyr Phe Pro Asp Gly Glu Pro Tyr Thr Thr Arg
               585                 590                 595
   Gly Leu Val Gly Leu Lys Asp Gly Glu Met Lys Asn Phe Ser Ser Met
           600                 605                 610
   Ser Pro Leu Ala Lys Gly Val Ser Val Tyr Phe Asp Gly Glu Gly Ile
       615                 620                 625
   His Phe Ile Val Lys Gly Asn Leu Asp Arg Phe Glu Val Ser Ile Trp
   630                 635                 640                 645
   Glu Lys Asp Glu Arg Val Gly Asn Thr Phe Thr Arg Leu Gln Glu Lys
                   650                 655                 660
   Pro Asp Glu Leu Ser Tyr Phe Met Phe Pro Phe Ser Arg Asp Ser Val
               665                 670                 675
   Gly Leu Leu Ile Thr Lys His Val Val Tyr Glu Asn Gly Lys Ala Glu
   680 685 690
   Ile Tyr Gly Ala Thr Asp Tyr Glu Lys Ser Glu Lys Leu Gly Glu Ala
   695 700 705
   Thr Val Lys Asn Thr Ser Glu Gly Ile Glu Val Val Leu Pro Phe Asp
   710 715 720 725
   Tyr Ile Glu Asn Pro Ser Asp Phe Tyr Phe Ala Val Ser Thr Val Lys
   730 735 740
   Asp Gly Asp Leu Glu Val Ile Ser Thr Pro Val Glu Leu Lys Leu Pro
   745 750 755
   Thr Glu Val Lys Gly Val Val Ile Ala Asp Ile Thr Asp Pro Glu Gly
   760 765 770
   Asp Asp His Gly Pro Gly Asn Tyr Thr Tyr Pro Thr Asp Lys Val Phe
   775 780 785
   Lys Pro Gly Val Phe Asp Leu Leu Arg Phe Arg Met Leu Glu Gln Thr
   790 795 800 805
   Glu Ser Tyr Val Met Glu Phe Tyr Phe Lys Asp Leu Gly Gly Asn Pro
   810 815 820
   Trp Asn Gly Pro Asn Gly Phe Ser Leu Gln Ile Ile Glu Val Tyr Leu
   825 830 835
   Asp Phe Lys Asp Gly Gly Asn Ser Ser Ala Ile Lys Met Phe Pro Asp
   840 845 850
   Gly Pro Gly Ala Asn Val Asn Leu Asp Pro Glu His Pro Trp Asp Val
   855 860 865
   Ala Phe Arg Ile Ala Gly Trp Asp Tyr Gly Asn Leu Ile Ile Leu Pro
   870 875 880 885
   Asn Gly Thr Ala Ile Gln Gly Glu Met Gln Ile Ser Ala Asp Pro Val
   890 895 900
   Lys Asn Ala Ile Ile Val Lys Val Pro Lys Lys Tyr Ile Ala Ile Asn
   905 910 915
   Glu Asp Tyr Gly Leu Trp Gly Asp Val Leu Val Gly Ser Gln Asp Gly
   920 925 930
   Tyr Gly Pro Asp Lys Trp Arg Thr Ala Ala Val Asp Ala Glu Gln Trp
   935 940 945
   Lys Leu Gly Gly Ala Asp Pro Gln Ala Val Ile Asn Gly Val Ala Pro
   950 955 960 965
   Arg Val Ile Asp Glu Leu Val Pro Gln Gly Phe Glu Pro Thr Gln Glu
   970 975 980
   Glu Gln Leu Ser Ser Tyr Asp Ala Asn Asp Met Lys Leu Ala Thr Val
   985 990 995
   Lys Ala Leu Leu Leu Leu Lys Gln Gly Ile Val Val Thr Asp Pro
   1000 1005 1010
   Glu Gly Asp Asp His Gly Pro Gly Thr Tyr Thr Tyr Pro Thr Asp
   1015 1020 1025
   Lys Val Phe Lys Pro Gly Val Phe Asp Leu Leu Lys Phe Lys Val
   1030 1035 1040
   Thr Glu Gly Ser Asp Asp Trp Thr Leu Glu Phe His Phe Lys Asp
   1045 1050 1055
   Leu Gly Gly Asn Pro Trp Asn Gly Pro Asn Gly Phe Ser Leu Gln
   1060 1065 1070
   Ile Ile Glu Val Tyr Phe Asp Phe Lys Glu Gly Gly Asn Val Ser
   1075 1080 1085
   Ala Ile Lys Met Phe Pro Asp Gly Pro Gly Ser Asn Val Arg Leu
   1090 1095 1100
   Asp Pro Asn His Pro Trp Asp Leu Ala Leu Arg Ile Ala Gly Trp
   1105 1110 1115
   Asp Tyr Gly Asn Leu Ile Ile Leu Pro Asp Gly Thr Ala Tyr Gln
   1120 1125 1130
   Gly Glu Met Gln Ile Ser Ala Asp Pro Val Lys Asn Ala Ile Ile
   1135 1140 1145
   Val Lys Val Pro Lys Lys Tyr Leu Asn Ile Ser Asp Tyr Gly Leu
   1150 1155 1160
   Tyr Thr Ala Val Ile Val Gly Ser Gln Asp Gly Tyr Gly Pro Asp
   1165 1170 1175
   Lys Trp Arg Pro Val Ala Ala Glu Ala Glu Gln Trp Lys Leu Gly
   1180 1185 1190
   Gly Ala Asp  Pro Gln Ala Val Ile  Asp Asn Leu Val Pro  Arg Val
         1195                 1200                 1205
   Val Asp Glu  Leu Val Pro Glu Gly  Phe Lys Pro Thr Gln  Glu Glu
         1210                 1215                 1220
   Gln Leu Ser  Ser Tyr Asp Leu Glu  Lys Lys Thr Leu Ala  Thr Val
         1225                 1230                 1235
   Leu Met Val  Pro Leu Val Asn Gly  Thr Gly Gly Glu Glu  Pro Thr
         1240                 1245                 1250
   Pro Thr Glu  Ser Pro Thr Glu Thr  Thr Thr Thr Thr Pro  Ser Glu
         1255                 1260                 1265
   Thr Thr Thr  Thr Thr Ser Thr Thr  Thr Gly Pro Ser Ser  Thr Thr
         1270                 1275                 1280
   Thr Ser Thr  Pro Gly Gly Gly Ile  Cys Gly Pro Gly Ile  Ile Ala
         1285                 1290                 1295
   Gly Leu Ala  Leu Ile Pro Leu Leu  Leu Lys Arg Arg Asn
         1300                 1305                 1310
<210> 12
   <211> 809
   <212> PRT
   <213> Artificial Sequence
<220>
   <223> Hybrid pullulanase of Thermoccus hydrothermalis and Thermococ litoralis
<220>
   <221> SIGNAL,
   <222> (1)..(27)
<220>
   <221> mat_peptide
   <222> (28)..(809)
<400> 12
   Met Lys Lys Pro Leu Gly Lys Ile Val Ala Ser Thr Ala Leu Leu Ile
           -25                 -20                 -15
   Ser Val Ala Phe Ser Ser Ser Ile Ala Ser Ala Glu Glu Pro Lys Pro
       -10                 -5              -1  1               5
   Leu Asn Val Ile Ile Val Trp His Gln His Gln Pro Tyr Tyr Tyr Asp
                   10                  15                  20
   Pro Ile Gln Asp Ile Tyr Thr Arg Pro Trp Val Arg Leu His Ala Ala
               25                  30                  35
   Asn Asn Tyr Trp Lys Met Ala Asn Tyr Leu Ser Lys Tyr Pro Asp Val
           40                  45                  50
   His Val Ala Ile Asp Leu Ser Gly Ser Leu Ile Ala Gln Leu Ala Asp
       55                  60                  65
   Tyr Met Asn Gly Lys Lys Asp Thr Tyr Gln Ile Val Thr Glu Lys Ile
   70                  75                  80                  85
   Ala Asn Gly Glu Pro Leu Thr Leu Glu Asp Lys Trp Phe Met Leu Gln
                   90                  95                  100
   Ala Pro Gly Gly Phe Phe Asp His Thr Ile Pro Trp Asn Gly Glu Pro
               105                 110                 115
   Val Ala Asp Glu Asn Gly Asn Pro Tyr Arg Glu Gln Trp Asp Arg Tyr
           120                 125                 130
   Ala Glu Leu Lys Asp Lys Arg Asn Asn Ala Phe Lys Lys Tyr Ala Asn
       135                 140                 145
   Leu Pro Leu Asn Glu Gln Lys Val Lys Ile Thr Ala Glu Phe Thr Glu
   150                 155                 160                 165
   Gln Asp Tyr Ile Asp Leu Ala Val Leu Phe Asn Leu Ala Trp Ile Asp
                   170                 175                 180
   Tyr Asn Tyr Ile Ile Asn Thr Pro Glu Leu Lys Ala Leu Tyr Asp Lys
               185                 190                 195
   Val Asp Val Gly Gly Tyr Thr Lys Glu Asp Val Ala Thr Val Leu Lys
           200                 205                 210
   His Gln Met Trp Leu Leu Asn His Thr Phe Glu Glu His Glu Lys Ile
       215                 220                 225
   Asn Tyr Leu Leu Gly Asn Gly Asn Val Glu Val Thr Val Val Pro Tyr
   230                 235                 240                 245
   Ala His Pro Ile Gly Pro Leu Leu Asn Asp Phe Gly Trp Tyr Glu Asp
                   250                 255                 260
   Phe Asp Ala His Val Lys Lys Ala His Glu Leu Tyr Lys Lys Tyr Leu
               265                 270                 275
   Gly Asp Asn Arg Val Glu Pro Gln Gly Gly Trp Ala Ala Glu Ser Ala
           280                 285                 290
   Leu Asn Asp Lys Thr Leu Glu Ile Leu Thr Asn Asn Gly Trp Lys Trp
       295                 300                 305
   Val Met Thr Asp Gln Met Val Leu Asp Ile Leu Gly Ile Pro Asn Thr
   310                 315                 320                 325
   Val Glu Asn Tyr Tyr Lys Pro Trp Val Ala Glu Phe Asn Gly Lys Lys
                   330                 335                 340
   Ile Tyr Leu Phe Pro Arg Asn His Asp Leu Ser Asp Arg Val Gly Phe
               345                 350                 355
   Arg Tyr Ser Gly Met Asn Gln Tyr Gln Ala Val Glu Asp Phe Val Asn
           360                 365                 370
   Glu Leu Leu Lys Val Gln Lys Glu Asn Tyr Asp Gly Ser Leu Val Tyr
       375                 380                 385
   Val Val Thr Leu Asp Gly Glu Asn Pro Trp Glu His Tyr Pro Phe Asp
   390                 395                 400                 405
   Gly Lys Ile Phe Leu Glu Glu Leu Tyr Lys Lys Leu Thr Glu Leu Gln
                   410                 415                 420
   Lys Gln Gly Leu Ile Arg Thr Val Thr Pro Ser Glu Tyr Ile Gln Met
               425                 430                 435
   Tyr Gly Asp Lys Ala Asn Lys Leu Thr Pro Arg Met Met Glu Arg Leu
           440                 445                 450
   Asp Leu Thr Gly Asp Asn Val Asn Ala Leu Leu Lys Ala Gln Ser Leu
       455                 460                 465
   Gly Glu Leu Tyr Asp Met Thr Gly Val Lys Glu Glu Met Gln Trp Pro
   470                 475                 480                 485
   Glu Ser Ser Trp Ile Asp Gly Thr Leu Ser Thr Trp Ile Gly Glu Pro
                   490                 495                 500
   Gln Glu Asn Tyr Gly Trp Tyr Trp Leu Tyr Met Ala Arg Lys Ala Leu
               505                 510                 515
   Met Glu Asn Lys Asp Lys Met Ser Gln Ala Asp Trp Glu Lys Ala Tyr
           520                 525                 530
   Glu Tyr Leu Leu Arg Ala Glu Ala Ser Asp Trp Phe Trp Trp Tyr Gly
       535                 540                 545
   Ser Asp Gln Asp Ser Gly Gln Asp Tyr Thr Phe Asp Arg Tyr Leu Lys
   550                 555                 560                 565
   Thr Tyr Leu Tyr Glu Met Tyr Lys Leu Ala Gly Val Glu Pro Pro Ser
                   570                 575                 580
   Tyr Leu Phe Gly Asn Tyr Phe Pro Asp Gly Glu Pro Tyr Thr Thr Arg
               585                 590                 595
   Gly Leu Val Gly Leu Lys Asp Gly Glu Met Lys Asn Phe Ser Ser Met
           600                 605                 610
   Ser Pro Leu Ala Lys Gly Val Ser Val Tyr Phe Asp Gly Glu Gly Ile
       615                 620                 625
   His Phe Ile Val Lys Gly Asn Leu Asp Arg Phe Glu Val Ser Ile Trp
   630                 635                 640                 645
   Glu Lys Asp Glu Arg Val Gly Asn Thr Phe Thr Arg Leu Gln Glu Lys
                   650                 655                 660
   Pro Asp Glu Leu Ser Tyr Phe Met Phe Pro Phe Ser Arg Asp Ser Val
               665                 670                 675
   Gly Leu Leu Ile Thr Lys His Val Val Tyr Glu Asn Gly Lys Ala Glu
           680                 685                 690
   Ile Tyr Gly Ala Thr Asp Tyr Glu Lys Ser Glu Lys Leu Gly Glu Ala
       695                 700                 705
   Thr Val Lys Asn Thr Ser Glu Gly Ile Glu Val Val Leu Pro Phe Asp
   710                 715                 720                 725
   Tyr Ile Glu Asn Pro Ser Asp Phe Tyr Phe Ala Val Ser Thr Val Lys
                   730                 735                 740
   Asp Gly Asp Leu Glu Val Ile Ser Thr Pro Val Glu Leu Lys Leu Pro
               745                 750                 755
   Thr Glu Val Lys Gly Val Val Ile Ala Asp Ile Thr Asp Pro Glu Gly
           760                 765                 770
   Asp Asp His Gly Pro Gly Asn Tyr Thr
       775                 780